# EUROPÄISCHE PATENTSCHRIFT

(11) **EP 1 349 826 B1**
(45) Veröffentlichungstag und Bekanntmachung des Hinweises auf die Patenterteilung: **27.06.2012**
(21) Anmeldenummer: 02703544.3
(22) Anmeldetag: 14.01.2002
(51) Int. Cl.: C07C 51/43, B01D 3/00

(54) **KONTINUIERLICHES VERFAHREN ZUR HERSTELLUNG UND AUFREINIGUNG VON (METH) ACRYLSÄURE**
CONTINUOUS METHOD FOR THE PRODUCTION AND PURIFICATION OF (METH)ACRYLIC ACID
PROCEDE CONTINU POUR LA PRODUCTION ET LA PURIFICATION D'ACIDE (METH)ACRYLIQUE

(30) Priorität: 12.01.2001 DE 10101160; 06.10.2001 DE 10149353
(43) Veröffentlichungstag der Anmeldung: 08.10.2003
(73) Patentinhaber: Evonik Stockhausen GmbH, 47805 Krefeld (DE)
(72) Erfinder: NORDHOFF, Stefan, 45657 Recklinghausen (DE); KOBUS, Axel, 44797 Bochum (DE); GROSS, Stephan, 63505 Langenselbold (DE); LAUSCH, Hans-Rolf, Luling, LA 70070 (US); BUB, Günther, 45772 Marl (DE); BALDUF, Torsten, 45772 Marl (DE); FORNIKA, Roland, 48249 Dülmen (DE)
(74) Vertreter: Lang, Arne
(86) Internationale Anmeldenummer: PCT/EP2002/000266
(87) Internationale Veröffentlichungsnummer: WO 2002/055469

(56) Entgegenhaltungen:
- WO-A-01/77056
- DE-A- 19 833 049
- US-A- 3 361 695
- US-A- 4 780 568

## Beschreibung

Die vorliegende Erfindung ist auf ein Verfahren zur Herstellung von sehr reiner (Meth)Acrylsäure, eine Vorrichtung zur Herstellung von sehr reiner (Meth)Acrylsäure, (Meth)Acrylsäure, erhältlich aus diesem Verfahren, deren Verwendung und diese beinhaltende Stoffe, sowie die Verwendung der Vorrichtung zur Herstellung von (Meth)Acrylsäure gerichtet. Insbesondere besteht das Verfahren aus zwei Verfahrensschritten, die im einzelnen das Kristallisieren und Waschen der kristallisierten (Meth)Acrylsäure umfassen.

"(Meth)Acrylsäure" wird in diesem Text für die Verbindungen mit dem Nomenklaturnamen "Methacrylsäure" und "Acrylsäure" verwendet. Von beiden Verbindung ist die Acrylsäure bevorzugt.

Die Aufarbeitung von (Meth)Acrylsäure zu Reinheiten von bis zu >99,9 Gew.-% ist für deren Einsatz in Polymeren häufig erwünscht. So wird beispielsweise im Hygienebereich im Falle von Superabsorbern auf Basis von Polyacrylaten gefordert, dass bestimmte Nebenprodukte nur unterhalb der Nachweisgrenze vorhanden sein dürfen.

Als eine Alternative zur Herstellung von hochreinen organischen Substanzen ist die Kristallisation zu nennen. Technisch kommen dabei insbesondere zwei Verfahren zur Anwendung, die Suspensionskristallisation und die Schichtkristallisation (Wintermantel et al., Chem. Ing. Tech. 1991, 63,881-891; Steiner et al, Chem. Ing. Tech. 1985, 57, 91-102).

Oft reicht allerdings eine Kristallisation alleine nicht aus, um Nebenprodukte hinreichend gut aus oder von den Kristallen zu entfernen, da Mikroeinschlüsse von Mutterlaugen oder Einbau von Verunreinigungen an Kristallfehlstellen etc. unter endlichen Kristallwachstumsbedingungen nicht auszuschließen sind. Auch das Anhaften von Mutterlauge auf dem Kristall kann die Reinheit der Produkte verschlechtern.

Aus diesem Grund werden die erzeugten Kristalle häufig nach der Trennung von der Mutterlauge mit Waschflüssigkeiten gewaschen und/oder die Kristalle werden einem Schwitzprozeß unterzogen, bei dem Verunreinigungen jeglicher Art ggf. abgereichert werden können. Kontinuierlich kann ein solcher Prozeß in sogenannten Waschkolonnen durchgeführt werden. Eine Übersicht bietet hier die Dissertation von Poschmann (Zur Suspensionskristallisation organischer Schmelzen und Nachbehandlung der Kristalle durch Schwitzen und Waschen, Diss. Uni. Bremen, Shaker Verlag, Aachen 1996).

Die EP0616998 offenbart ein Verfahren zur Herstellung von >99,9 Gew.-%iger Acrylsäure ausgehend von vorgereinigtem Produkt mit einem Acrylsäuregehalt von 97,771 Gew.-%. Der Reinigungseffekt wird durch ein Zusammenwirken von dynamischen und statischen Kristallisationsverfahren erreicht. Als finales Kristallisationsorgan findet hier eine sogenannte Fallfilmkristallisation Anwendung. Das Betreiben einer derartigen Anlage ist nur diskontinuierlich möglich und bedingt durch die vielen Prozeßzyklen, die zum Erhalt der entsprechenden Reinheiten notwendig sind, einen hohen apparativen wie logistischen Aufwand und einen vergleichsweise hohen Energieaufwand.

Aus WO99/14181 ist bekannt, Roh-(Meth)Acrylsäure zur Reinigung in einem ersten Schritt zu kristallisieren und in einem zweiten Schritt ggf. mittels Waschkolonnen aufzuarbeiten. Das dort offenbarte Verfahren geht direkt von den Kondensationsprodukten der katalytischen Gasphasenoxidation zur Erzeugung von (Meth)Acrylsäure aus. Bei diesem Verfahren wird beschrieben, dass die nach dem Waschen und Abtrennen der Kristalle entstehende Mutterlauge in die Kondensationsstufe zurückgeführt wird. Mittels dieses Verfahrens erhielt man aus 90,972 Gew.-%iger Acrylsäure ein Produkt mit einer Reinheit von 98,8816 Gew.-%. Dies ist für manche technischen Anwendungen jedoch nicht ausreichend. So bildet gerade der Gehalt an Inhibitoren und aldehyden in der reinen (Meth)Acrylsäure eine kritische Größe, die bei Überschreitung z.B. in den nachgeschalteten Polymerisationsverfahren für Nachteile sorgt.

Von Nienrood et al. wurde beschrieben, dass Acrylsäure durch Suspensionskristallisation und anschließende Behandlung in einer hydraulischen waschkolonne gut aufgereinigt werden kann (sogenanntes TNO-Verfahren; Proc. Bremer International Workshop on Industrial Crystallization, Bremen, 1994, Hrsg.: J. Ulrich, S.4-11; Purification Potential of Suspension Growth Melt Crystallization, Proc. 4th International Workshop on Crystal Growth of Organic Materials, Bremen, 1997, Hrsg.: J. Ulrich, Aachen Shaker Verlag, S.139-145). Die bei diesen Untersuchungen eingesetzte Acrylsäure wurde von Aldrich bezogen und hatte eine Feinheit von 99,75 Gew.-%. Sie ließ sich mittels dieses Verfahrens auf eine Reinheit von 99,97 Gew.-% aufreinigen. Nicht offenbart wurde jedoch der Einsatz von Acrylsäure mit geringeren Reinheiten.

In der US 4780568 wird ein Verfahren beschrieben, bei dem ein Stoffgemisch eingesetzt wird, welches durch oxidative Dehydrierung von iso-Buttersäure erhalten wird und im wesentlichen frei von Wasser ist. Nachteilig ist hierbei der hohe Anteil an iso-Buttersäure im Endprodukt in einer Menge von 10 bis 15 Gew. %.

Die DE 198 33 049 betrifft die Herstellung von reiner Acrylsäure, durch ein Verfahren, bei dem in einem Schritt ein Vakuum angelegt wird. Nachteilig hierbei sind die hohen wirtschaftlichen Kosten für den Vakuumschritt.

Allgemein liegt der vorliegenden Erfindung die Aufgabe zu Grunde, die sich aus dem Stand der Technik ergebenden Nachteile durch die Bereitstellung geeigneter technischer Lehren zu überwinden.

Aufgabe der vorliegenden Erfindung war deshalb die Angabe eines Verfahrens zur Erzeugung hochreiner (Meth)Acrylsäure aus einem verunreinigten Roh-(Meth)Acrylsäurestrom aus einem verfahren zur Herstellung von (Meth)ACrylsäure. Dabei sollte das Verfahren im technischen Maßstab gut anwendbar und deshalb vom ökonomischen wie ökologischen Standpunkt den Verfahren des Standes der Technik überlegen sein. Insbesondere sind in diesem Zusammenhang ein hervorragendes Aufreinigungsvermögen, bei Einhaltung hoher ökologischer und ökonomischer Anforderungen zu nennen.

Zudem lag eine weitere der Erfindung zu Grunde liegende Aufgabe darin, eine Vorrichtung zur Erzeugung hochreiner (Meth)Acrylsäure zur Verfügung zu stellen, die eine Aufreinigung von verunreinigter (Meth)Acrylsäure zu höchster Reinheit bei einem geringen Energieaufwand und störungsfreien und umweltschonenden Betrieb ermöglicht.

Ferner lag der Erfindung die Aufgabe zu Grunde, eine Verfahren und eine vorrichtung zur Verfügung zu stellen, wobei bei der Herstellung und insbesondere bei der Aufreinigung von (Meth)Acrylsäure die Gefahr, das es zu unkontrollierter Polymerisation von (Meth)Acrylsäure kommt, verringert wird.

Schließlich war es Aufgabe, eine weitere Verwendung der Vorrichtung zur Herstellung der (Meth)Acrylsäure bzw. eine ihrer Komponenten anzugeben.

Diese Aufgaben werden gelöst durch ein Verfahren mit den Merkmalen gemäß Anspruch 1; durch eine Vorrichtung mit den Merkmalen gemäß Ansprüch 7; durch eine (Meth)Acrylsäure mit den Merkmalen gemäß Anspruch 12; durch eine Verwendung mit den Merkmalen gemäß Anspruch 15. Weitere vorteilhafte Ausführungsformen und Weiterbildungen sind Gegenstand der jeweils abhängigen Ansprüche, die jeweils einzeln angewandt oder beliebig mit einander kombiniert werden können.

Das erfindungsgemäße Verfahren zur Aufreinigung von (Meth)Acrylsäure enthält eine Verfahrensstufe, welche folgende verfahrensschritte umfasst:
a) (Meth)Acrylsäure wird aus einer Mutterlauge auskristallisiert;
b) kristallisierte (Meth)Acrylsäure wird von der Mutterlauge abgetrennt;
c) zumindest ein Teil der abgetrennten (Meth)Acrylsäurekristalle wird aufgeschmolzen; und
d) der aufgeschmolzene Teil wird dem Schritt a) oder dem Schritt b) zumindest teilweise zurückgeführt.

Mit diesem Verfahren gelangt man in überraschender Weise, dafür aber nicht minder vorteilhaft, zu hoch reinen Produkten, die sich auch für den Einsatz in Polymeren, z.B. für den Hygienebereich, eignen.

Das Verfahren arbeitet vorzugsweise kontinuierlich. Zur Abtrennung der (Meth)Acrylsäure von der Mutterlauge wird vorteilhafterweise eine Waschkolonne verwendet. Dazu weist die Waschkolonne einen Trennbereich auf, in dem die (Meth)Acrylsäurekristalle gewaschen werden. Für den erfolgreichen Betrieb einer Waschkolonne ist es vorteilhaft, dass die zu waschenden Kristalle hart genug sind und eine bestimmte enge Größenverteilung aufweisen, um eine entsprechende Porösität und Stabilität des entstehenden gepackten oder ungepackten Filterbettes zu gewährleisten. Dass (Meth)Acrylsäure selbst mit technischen Qualitäten von <99,5 Gew.-% zur Ausbildung der geforderten Kristalleigenschaften im Stande ist, war vorher nicht bekannt. Es ist mithin diese Eigenschaft der (Meth)Acrylsäure, die es erlaubt, das erfindungsgemäße Verfahren mit einer Waschkolonne zu betreiben und gleichzeitig die entstehende Mutterlauge ggf. direkt zumindest teilweise in die Kristallisation zurückzuführen, ohne die Kristallqualität durch Akkumulation der Nebenprodukte derart negativ zu beeinflussen, dass das erfindungsgemäße Verfahren nicht mehr erfolgreich ausgeführt werden kann. Dies wird durch den Stand der Technik in keiner Weise nahegelegt.

Weiterhin ist es bevorzugt, dass das erfindungsgemäße Verfahren durch einen Kristallisationsaufwand (Wellinghoff, Wintermantel, CIT 63 (1991) 9, S. 805 Kap. 3.2.1) von 1 bis 4,5, vorzugsweise weniger 1 bis 3,5 und besonders bevorzugt von 1 bis 2,5 sowie darüber hinaus bevorzugt von 1 bis 1,5 gekennzeichnet ist.

Das erfindungsgemäße Verfahren kann mit verunreinigter Roh-(Meth)Acrylsäure betrieben werden, die <99,5 Gew.-% (Meth)Acrylsäure aufweist. Bevorzugt hat die eingesetzte Roh-(Meth)Acrylsäure eine Reinheit von 50 Gew.-% bis 95 Gew.-%, vorzugsweise 75 Gew.-% bis 90 Gew.-% an (Meth)Acrylsäure.

Verfahren zur Aufreinigung von (Meth)acrylsäure mit einer Verfahrensstufe, welche folgende Verfahrensschritte umfasst:
a) (Meth)acrylsäure wird aus einer Mutterlauge auskristallisiert;
b) kristallisierte (Meth)acrylsäure wird von der Mutterlauge abgetrennt;
c) zumindest ein Teil der abgetrennten (Meth)acrylsäurekristalle wird aufgeschmolzen; und
d) der aufgeschmolzene Teil wird dem Schritt a) oder dem Schritt b) zumindest teilweise zurückgeführt,
wobei die im Verfahrensschritt a) eingesetzte (Meth)acrylsäure ein Roh-(Meth)acrylsäurestrom ist, der erhalten wird durch die Destillation einer wässrigen (Meth)acrylsäurelösung in einer Destillationsvorrichtung, wobei die wässrige (Meth)acrylsäurelösung durch Kondensation des Gemisches einer katalytischen Gasphasenoxidation von Isobuten, Isobutan, tert-Butanol, iso-Butyraldehyd, Methacrolein oder Meth-tert.-Butylether mit Sauerstoff in einem Quenchtower erhalten wird.

Ganz besonders bevorzugt kann man das kondensierte Gemisch einer katalytischen Gasphasenoxidation zur Herstellung von (Meth)Acrylsäure direkt in den Schritt a) einsetzen. Dazu wird (Meth)Acrylsäure in einem Reaktor generiert, dann wird sie in einem Quenchtower in wässerige Lösung gebracht, anschließend wird die (Meth)Acrylsäure in einer Destillationsvorrichtung destilliert. Der so hergestellte Roh-(Meth)Acrylsäurestrom wird der Aufreinigungsvorrichtung zugeführt. Das Verfahren erlaubt die Herstellung sehr reiner (Meth)Acrylsäure aus vergleichsweise verunreinigter Roh-(Meth)Acrylsäure. In der Folge kann die Destillationschritt schonender erfolgen, wodurch eine vorzeitige Polymerisation reduziert und die Qualität der (Meth)Acrylsäure gesteigert wird.

Bei der Kristallisation sind als Kristallisationsmittel solche zu verwenden, die es gestatten, den erfindungsgemäßen Aufreinigungsprozeß kontinuierlich zu gestalten. Vorzugsweise kommt die Suspensionskristallisation zum Einsatz. Diese kann vorteilhafterweise in einem Rührkesselkristallisator, Kratzkristallisator, Kühlscheibenkristallisator, Kristallisierschnecke, Trommelkristallisator, Rohrbündelkristallisator o. dgl. durchgeführt werden. Insbesondere können die in WO99/14181 genannten Kristallisationsvarianten für den genannten Zweck herangezogen werden. Von besonderem Vorteil sind hier wiederum solche Kristallisatoren, die kontinuierlich betrieben werden können. Vorzugsweise sind dies die Kühlscheibenkristallisatoren oder der Kratzkühler (Diss. Poschmann, S. 14). Ganz besonders bevorzugt wird zur Kristallisation ein Kratzkühler eingesetzt.

Im Prinzip kann für das gegenständliche Verfahren jedwede Waschkolonne eingesetzt werden, die die kontinuierliche Fahrweise des erfindungsgemäßen Reinigungsprozesses zuläßt. Bei einer üblichen Ausführungsform wird die Suspension in einer hydraulischen Waschkolonne im oberen Teil der Kolonne aufgegeben; die flüssige Phase (Mutterlauge) wird über ein Filter aus der Kolonne abgezogen, wodurch sich ein dichtgepacktes Kristallbett bildet. Das Kristallbett wird von der flüssigen Phase in Richtung des Bodens der Kolonne durchströmt und durch den Strömungswiderstand nach unten gedrückt. Am Boden der Kolonne befindet sich eine bewegte, vorzugsweise rotierende Kratzvorrichtung oder Kratzer, die aus dem dichtgepackten Kristallbett und der am unteren Teil der Waschkolonne eingebrachten Waschschmelze wieder eine Suspension erzeugt. Diese Suspension wird vorzugsweise durch einen Wärmetauscher gepumpt und aufgeschmolzen. Ein Teil der Schmelze kann z.B. als Waschschmelze dienen; diese wird dann in die Kolonne zurückgepumpt und wäscht vorzugsweise das in entgegengesetzter Richtung wandernde Kristallbett aus, d.h. die kristallisierte (Meth)Acrylsäure wird im Gegenstrom von der zurückgeführten (Meth)Acrylsäure gewaschen. Die Waschschmelze bewirkt einerseits ein Waschen der Kristalle, andererseits kristallisiert die Schmelze auf den Kristallen zumindest teilweise aus. Die freiwerdende Kristallisationsenthalphie erwärmt das Kristallbett im Waschbereich der Kolonne. Dadurch wird ein dem Schwitzen der Kristalle analoger Reinigungseffekt erzielt. Damit wird eine Aufreinigung zum einen durch das Waschen der Oberfläche der (Meth)Acrylsäurekristalle mit aufgeschmolzener - und damit bereits gereinigter- (Meth)Acrylsäure bewirkt, zum anderen wird durch die Kristallisation der aufgeschmolzenen, gereinigten (Meth)Acrylsäure auf den bereits vorhandenen (Meth)Acrylsäurekristallen ein Ausheilen bzw. Ausschwitzen von Verunreinigungen erreicht. Dieses erlaubt die hochreine Herstellung der (Meth)Acrylsäure.

In einer speziellen Ausgestaltung des erfindungsgemäßen Verfahrens ist der Rückführstrom, der sich aus der Zurückführung der aufgeschmolzenen (Meth)Acrylsäure aus dem Schritt c) in den Schritt a) bzw. in den Schritt b) ergibt, größer als ein Feedstrom, der von außen kontinuierlich dem Schritt a) zugeführt wird.

Insbesondere ist der Rückführstrom mindestens doppelt, vorzugsweise mindestens zehnmal, so groß wie der Feedstrom. Durch den großen Rückführstrom wird sichergestellt, dass die thermische Belastung der (Meth)Acrylsäure am Aufheizer verringert wird.

Zur Impfung der zu kristallisierenden (Meth)Acrylsäure ist es vorteilhaft die abgetrennte, kristallisierte (Meth)Acrylsäure aus Schritt b) zumindest teilweise in den Schritt a) zuführen. Die zurückgeführten (Meth)Acrylsäurekristalle erleichtern das Kristallwachstum im Schritt a) und unterstützen so die Trennung der (Meth)Acrylsäure von der Mutterlauge.

Grundsätzlich ist aufgrund energetischer Überlegungen ein einstufiges Aufreinigungsverfahren mit nur einer Verfahrensstufe besonders vorteilhaft und damit besonders bevorzugt. Unter Umständen ist jedoch ein zweistufiges Aufreinigungsverfahren sinnvoll.

Zur Steigerung der Ausbeute ist es zweckmäßig, die nach Schritt b) abgetrennte Mutterlauge zumindest teilweise in den Schritt a) zurückzuführen. In der Mutterlauge verbliebene (Meth)Acrylsäure kann so mit geeigneten räumlichen Temperaturprofilen, vorzugsweise bis zum thermodynamischen Limit (z.B. Eutektikum) weiter auskristallisiert werden.

In einer vorteilhaften Gruppe von Ausgestaltungen weist das erfindungsgemäße Verfahren mindestens zwei Verfahrensstufen auf, die jeweils die Schritte a) bis d) aufweisen, wobei mindestens eines der folgenden Merkmale (α1) bis (α4) erfüllt ist:
(α1) abgetrennte, d.h. in diesem Fall insbesondere kristalline und/oder aufgeschmolzene, (Meth)Acrylsäure aus einer ersten Verfahrensstufe wird zumindest teilweise einer zweiten Verfahrensstufe zugeführt;
(α2) abgetrennte, d.h. in diesem Fall insbesondere kristalline und/oder aufgeschmolzene, (Meth)Acrylsäure aus einer zweiten Verfahrensstufe wird zumindest teilweise einer ersten Verfahrensstufe zugeführt;
(α3) Mutterlauge, d.h. insbesondere nach dem Schritt b) abgetrennte Mutterlauge, einer ersten Verfahrensstufe wird zumindest teilweise einer zweiten Verfahrensstufe zugeführt;
(α4) Mutterlauge, d.h. insbesondere nach dem Schritt b) abgetrennte Mutterlauge, einer zweiten Verfahrensstufe wird zumindest teilweise einer ersten Verfahrensstufe zugeführt.

Aus dieser Gruppe vorteilhafter Ausgestaltungen mit mindestens einem der Merkmale (α1) bis (α4) der Erfindung sind die Ausgestaltungen bevorzugt, bei den mindestens eines der der folgenden Merkmale (β1) und (β6) erfüllt ist:
(β1) kristalline (Meth) Acrylsäure aus der ersten Verfahrenstufe wird zumindest einem der Schritte a) und b) der zweiten Verfahrenstufe zugeführt;
(β2) aufgeschmolzene (Meth)Acrylsäure aus der ersten Verfahrenstufe wird zumindest einem der Schritte a) und b) der zweiten Verfahrenstufe zugeführt;
(β3) kristalline (Meth)Acrylsäure aus der zweiten Verfahrensstufe wird zumindest einem der Schritte a), b) und c) der ersten Verfahrensstufe zugeführt;
(β4) aufgeschmolzene (Meth)Acrylsäure aus der zweiten Verfahrenstufe wird zumindest einem der Schritte a), b) und c) der ersten Verfahrenstufe zugeführt;
(β5) die nach dem Schritt b) abgetrennte Mutterlauge der ersten Verfahrenstufe wird zumindest teilweise dem Schritt a) der zweiten Verfahrenstufe zugeführt;
(β6) die nach dem Schritt b) abgetrennte Mutterlauge der zweiten Verfahrenstufe wird zumindest teilweise dem Schritt a) der ersten Verfahrenstufe zugeführt.

Aus dieser Gruppe bevorzugter Ausführungsformen der Erfindung sind für den Fall, dass die zweite bzw. weitere Verfahrensstufe eine zusätzliche Aufreinigung der (Meth)Acrylsäure bewirkt, d.h. bereits in einer ersten Verfahrensstufe gereinigte (Meth)Acrylsäure in einer zweiten (weiteren) Verfahrensstufe weiter gereinigt wird, folgende Ausführungsformen besonders bevorzugt:
(1) zur Erzielung einer guten Reinheit der (Meth)Acrylsäure wird aufgeschmolzene (Meth)Acrylsäure aus der ersten Verfahrensstufe dem Schritt a) der zweiten Verfahrensstufe zugeführt;
(2) zur Erzielung einer guten Reinheit der (Meth)Acrylsäure wird kristalline (Meth)Acrylsäure aus der ersten Verfahrensstufe dem Schritt a) der zweiten Verfahrensstufe zugeführt;
(3) in gründliches Waschen der (Meth)Acrylsäure-kristalle der ersten Stufe zu ermöglichen, wird aufgeschmolzene (Meth)Acrylsäure aus der zweiten Verfahrensstufe dem Schritte b) der ersten Verfahrensstufe zugeführt; oder
(4) um besonders reine Impfkristalle zur Verfügung zu stellen wird kristalline (Meth)Acrylsäure aus der zweiten Verfahrensstufe dem Schritt a) der ersten Verfahrensstufe zugeführt.

Hierbei ganz besonders bevorzugt ist eine Kombination aus der Ausführungsformen (1) bzw. (2) und (3).

Aus der Gruppe bevorzugter Ausführungsformen der Erfindung sind für den Fall, dass die zweite Verfahrensstufe zur Steigerung der Ausbeute dient, folgende Ausführungsformen besonders bevorzugt:
(1) kristalline (Meth)Acrylsäure aus der ersten Verfahrensstufe werden als Impfkristalle zur Kristallisation dem Schritt a) der zweiten Verfahrensstufe zugeführt;
(2) zur Steigerung der Ausbeute wird die nach Schritt b) abgetrennte Mutterlauge der ersten Verfahrensstufe zumindest teilweise dem Schritt a) der zweiten Verfahrensstufe zugeführt;
(3) zur Steigerung der Reinheit wird aufgeschmolzene (Meth)Acrylsäure aus dem zweiten Verfahrensschritt dem Schritt a) der ersten Verfahrensstufe zugeführt; oder
(4) zur Minimierung des Energieaufwands wird kristalline (Meth)Acrylsäure aus dem zweiten Verfahrensschritt dem Schritt a) der ersten Verfahrensstufe zugeführt.

Hierbei ganz besonders bevorzugt ist eine Kombination aus den Ausführungsformen (2) und (4).

Vorteilhafterweise sind mindestens zwei verfahrensstufen in Reihe vorgesehen. In Reihe kann sich sowohl auf die abgetrennte (Meth)Acrylsäure, d.h. auf die kristalline oder auf die aufgeschmolzene (Meth)Acrylsäure, als auch auf die abgetrennte Mutterlauge beziehen.

Vorteilhaft ist es auch mindestens zwei Verfahrenstufen miteinander zu verschachteln. Hierdurch wird bewirkt, dass die Anzahl der für das Aufschmelzen notwendigen Aufschmelzer, die als Wärmetauscher bzw. Heizungen energieaufwendig im Betrieb sind, geringer ist als die Anzahl der Stufen. Beispielsweise kann eine einfache Verschachtelung von zwei Verfahrensstufen eine Ersparnis eines Aufschmelzers bedeuten. Hiermit wird der Betrieb bei gleicher Ausbeute und gleicher Reinheit deutlich kostengünstiger.

Die Anzahl der Verfahrensstufen richtet sich nach der mit dem Verfahren zu erzielenden Reinheit und Wirtschaftlichkeit. Grundsätzlich ist die erzielbare Reinheit von (Meth)Acrylsäure durch das thermodynamische Limit (z.B. Eutektikum) für eine Kristallisierbarkeit von (Meth)Acrylsäure aus der Mutterlauge begrenzt.

Eine spezielle Ausgestaltung des erfindungsgemäßen Verfahren ist gekennzeichnet durch folgende Merkmale (γ1) und (γ2) :
(γ1) Kristallisation von (Meth)Acrylsäure aus einem verunreinigten Roh-(Meth)Acrylsäurestrom aus einem Verfahren zur Herstellung von (Meth)Acrylsäure;
(γ2) Abtrennen der (Meth)Acrylsäurekristalle aus der Mutterlauge mittels einer Waschkolonne, wobei man die Mutterlauge aus Schritt (γ2) zumindest teilweise in den Schritt (γ1) zurückführt, wobei der Roh-(Meth)Acrylsäurestrom eine Reinheit von <99,5 Gew.-% an (Meth)ACrylsäure aufweist.

Die erfindungsgemäße Vorrichtung zur Herstellung von (Meth)Acrylsäure beinhaltet als fluidleitend miteinander verbundene Komponenten einen (Meth)Acrylsäure-Reaktor, einen Quenchtower, eine Destillationsvorrichtung und eine Aufreinigungsvorrichtung, wobei die Aufreinigungsvorrichtung eine Vorrichtungseinheit aufweist, welche die Merkmale (δ1) bis (δ4) umfasst:
(δ1) die Vorrichtungseinheit weist einen Kristallisationsbereich, einen Trennbereich, einen Aufschmelzer, und mindestens drei Führungen auf;
(δ2) der Kristallisationsbereich ist mit dem Trennbereich über eine erste Führung verbunden;
(δ3) der Trennbereich ist mit dem Aufschmelzer über eine zweite Führung verbunden;
(δ4) der Aufschmelzer ist mit dem Kristallisationsbereich über eine dritte Führung oder mit dem Trennbereich über eine vierte Führung verbunden.

Unter "fluidleitend" wird erfindungsgemäß verstanden, dass Gase oder Flüssigkeiten oder deren Mischungen durch entsprechende Leitungen geführt werden. Hierzu lassen sich insbesondere Rohleitungen, Pumpen und der gleichen einsetzen.

Eine Vorrichtung zur Herstellung von Acrylsäure weist in dem Bereich, der einen (Meth)Acrylsäurereaktor und einen Quenchtower aufweist, vorzugsweise folgenden Aufbau bei der Synthese von Acrylsäure auf: Propylen und ggf. weiter Inertgase wie Stickstoff oder Verbrennungsgase wie CO₂ oder Stickoxide werden in einen ersten Reaktor zur einer ersten katalytischen Oxidation über eine Eduktzufuhr, die in den ersten Reaktor mündet zugeleitet. Der erste Reaktor ist über eine weitere Leitung mit einem zweiten Reaktor verbunden, in den das Produkt der ersten katalytischen Oxidation aus dem ersten Reaktor für eine zweite katalytische Oxidation eingeleitet wird. Das Acrylsäure beinhaltende Produkt der zweiten katalytischen Oxidation wird über eine zwischen dem zweiten Reaktor und dem Quenchtower befindlichen Leitung der unteren Hälfte des Quenchtowers zugeführt. In dem Quenchtower wird das Produkt der zweiten katalytischen Oxidation mit Wasser in Kontakt gebracht, wobei das Wasser oberhalb der Zuführung des Produkts der zweiten katalytischen Oxidation in den eingespeist wird. Zum einen wird eine Acrylsäure und Wasser beinhaltende erste Phase unterhalb der Zuführung des Produkts der zweiten katalytischen Oxidation aus dem Quenchtower abgeführt. Diese erste Phase kann zumindest teilweise wieder oberhalb der Zuführung des Produkts der zweiten katalytischen Oxidation wieder in den Quenchtower zurückgeführt werden. Die nicht in den Quenchtower zurückgeführte erste Phase wird der Destillationsvorrichtung zugeführt, um beispielsweise einer azeotropen Trennung unterzogen zu werden, in der die Acrylsäure aufkonzentriert und gereinigt wird. Oberhalb der Rückführung der ersten Phase und unterhalb der Einspeisung von Wasser in den Quenchtower kann eine Acrylsäure und Wasser beinhaltende zweite Phase aus dem Quenchtower abgeführt werden. Diese zweite Phase kann genauso wie die erste Phase der Destillationsvorrichtung zugeführt werden, um beispielsweise einer azeotropen Trennung unterzogen zu werden, in der die Acrylsäure aufkonzentriert und gereinigt wird. Die aus dem Quenchtower abgeleiten Abgase können einer katalytischen Verbrennung zugeführt werden. Die Verbrennungsgase der katalytischen Verbrennung können als Inertgase in den ersten Reaktor eingespeist werden. Das bei der Aufkonzentration von Acrylsäure wiedergewonnene Wasser kann in den Quenchtower zurückgeführt werden. Weitere Einzelheiten zur Herstellung von Acrylsäure sind in DE 197 40 252 A1 offenbart, auf deren Inhalt hiermit als Teil dieser Offenbarung Bezug genommen wird.

Eine Vorrichtung zur Herstellung von Methacrylsäure weist den (Meth)Acrylsäurereaktor und einen Quenchtower bei der Synthese von Methacrylsäure durch katalytische Gasphasenreaktion von C4-Ausgangsverbindungen mit Sauerstoff auf. Besonders bevorzugt ist Methacrylsäure durch katalytische Gasphasenoxidation von Isobuten, Isobutan, tert.-Butanol, iso-Butyraldehyd, Methacrolein oder Meth-tert.-butylether erhältlich. Weitere Einzelheiten zur Herstellung von Acrylsäure sind in EP 0 092 097 B1, EP 0 058 927 und EP 0 0608 838 offenbart, auf deren Inhalt hiermit als Teil dieser Offenbarung Bezug genommen wird.

Die Aufreinigungsvorrichtung ist in der Lage aus einem vergleichsweise verunreinigten Roh-(Meth)Acrylsäurestrom von rund 85 Gew.-% sehr reine (Meth)Acrylsäure mit Reinheitsgraden von über 99,5 Gew.-% zu gewinnen. Es ist nach der Erfindung möglich, verunreinigte Roh- (Meth)Acrylsäurestrom mit 50 Gew.-% bis 95 Gew.-% (Meth)Acrylsäure, vorzugsweise 75 Gew.-% bis 90 Gew.-% (Meth)Acrylsäure effizient aufzureinigen. Diese effektive Aufreinigung erlaubt, die Vorreinigung des Roh-(Meth)Acrylsäurestroms mit Hilfe der Destillationsvorrichtung zu reduzieren, wodurch die thermische Belastung der (Meth)Acrylsäure reduziert wird. Hiermit wird die Qualität der (Meth)Acrylsäure verbessert.

Zur weiteren Steigerung der Reinheit der (Meth)Acrylsäure weist die Vorrichtungseinheit eine separate Reinigungsvorrichtung auf. Diese separate Reinigungsvorrichtung kann sowohl zur weitern Aufreinigung des Endprodukts als auch zur weiteren Aufreinigung der den Aufschmelzer verlassenden (Meth)Acrylsäure eingesetzt werden.

Zur verbesserten Kristallisation ist vorzugsweise der Trennbereich mit dem Kristallisationsbereich durch eine erste Rückführung für abgetrennte (Meth)Acrylsäure verbunden.

Zur Steigerung der Ausbeute ist vorteilhafterweise der Trennbereich mit dem Kristallisationsbereich durch eine zweite Rückführung für abgetrennte Mutterlauge verbunden ist.

Grundsätzlich ist aufgrund energetischer Überlegungen eine einstufige Aufreinigungsvorrichtung mit nur einer Vorrichtungseinheit besonders vorteilhaft und damit besonders bevorzugt. Unter Umständen ist jedoch eine zweistufige Aufreinigungsvorrichtung sinnvoll.

Zur Steigerung der Effektivität der Aufreinigung, insbesondere zur Steigerung der Reinheit und der Ausbeute, enthält die erfindungsgemäße Vorrichtung mindestens zwei Vorrichtungseinheiten gemäß den Merkmalen (δ1) bis (δ4), die durch mindestens eine verbindungsleitung verbunden sind, wobei die Verbindungsleitung eine Zuleitung oder eine Rückleitung ist, und wobei mindestens eines der folgenden Merkmale (ε1) bis (ε4) erfüllt ist:
(ε1) der Trennbereich einer ersten Vorrichtungseinheit ist über die Verbindungsleitung mit dem Kristallisationsbereich einer zweiten Vorrichtungseinheit verbunden;
(ε2) der Aufschmelzer einer ersten vorrichtungseinheit ist über die Verbindungsleitung mit dem Kristallisationsbereich einer zweiten Vorrichtungseinheit verbunden;
(ε3) der Trennbereich einer zweiten Vorrichtungseinheit ist über die Verbindungsleitung mit dem Kristallisationsbereich einer ersten Vorrichtungseinheit verbunden;
(ε4) der Aufschmelzer einer zweiten Vorrichtungseinheit ist über die Verbindungsleitung mit dem Kristallisationsbereich einer ersten Vorrichtungseinheit verbunden.

Hierbei können die Verbindungsleitungen sowohl Zu- wie auch Rückleitung sein.

Beispielsweise ist es zweckmäßig entsprechend des erfindungsgemäßen Verfahrens zur Steigerung der Ausbeute von dem Trennbereich der ersten Vorrichtungseinheit (erste Verfahrensstufe) eine Zuleitung zum Kristallisationsbereich der zweiten Vorrichtungseinheit (zweite Verfahrensstufe) vorzusehen. Eine Rückführung zwischen dem Trennbereich der zweiten Stufe und dem Kristallisationsbereich der ersten Stufe ist hingegen zur Bereitstellung von Impfkristallen zweckmäßig.

Damit ist es auf vorteilhafte Weise möglich, zur Verbesserung der Reinheit und der Ausbeute eine Reihenschaltung von mindestens zwei Vorrichtungseinheiten vorzusehen.

Durch eine Verschachtelung von mindestens zwei Vorrichtungseinheiten kann die Anzahl der erforderlichen Aufschmelzer verringert und damit der Energieaufwand zum Betrieb der Vorrichtung reduziert werden.

Bei dem erfindungsgemäßen Verfahren zur Aufreinigung von Acrylsäure herrschen in dem Trennbereich eine Temperatur im Bereich von -20 bis 20°C, vorzugsweise von -10 bis 13°C bei einem Druck von 1 bis 10 bar. Es ist bevorzugt, dass in dem unteren Bereich des Trennbereichs eine geringere Temperatur und ein geringerer Druck herrschen, als im oberen Bereich des Trennbereichs. Vorzugsweise herrschen in dem unteren Bereich des Trennbereichs -20 bis < 12°C bei einem Druck von 1 bis 2 bar. Im oberen Bereich des Trennbereichs herrscht eine Temperatur von mindestens 12°C und ein Druck von 1 bis 10 bar, vorzugsweise 3 bis 7 bar.

Bei dem erfindungsgemäßen Verfahren zur Aufreinigung von Acrylsäure herrschen in dem Kristallisationsbereich eine Temperatur im Bereich von -20 bis 20°C, vorzugsweise von - 12 bis 13°C bei einem Druck von 0,5 bis 10 bar, vorzugsweise 0,8 bis 2 bar.

Bei dem erfindungsgemäßen Verfahren zur Aufreinigung von Acrylsäure herrschen in dem Aufschmelzer eine Temperatur im Bereich von 10 bis 50°C, vorzugsweise von 11 bis 30°C bei einem Druck von 1 bis 10 bar, vorzugsweise 3 bis 7 bar.

Bei dem erfindungsgemäßen Verfahren zur Aufreinigung von Methacrylsäure herrschen in dem Trennbereich eine Temperatur im Bereich von -5 bis 30°C, vorzugsweise von -3 bis 20°C bei einem Druck von 1 bis 10 bar. Es ist bevorzugt, dass in dem unteren Bereich des Trennbereichs eine geringere Temperatur und ein geringerer Druck herrschen, als im oberen Bereich des Trennbereichs. Vorzugsweise herrschen in dem unteren Bereich des Trennbereichs -16 bis < 15°C bei einem Druck von 1 bis 2 bar. Im oberen Bereich des Trennbereichs herrscht eine Temperatur von mindestens 15°C und ein Druck von 1 bis 10 bar.

Bei dem erfindungsgemäßen Verfahren zur Aufreinigung von Acrylsäure herrschen in dem Kristallisationsbereich eine Temperatur im Bereich von -5 bis 30°C, vorzugsweise von -3 bis 20°C bei einem Druck von 1 bis 10 bar, vorzugsweise 1 bis 2 bar.

Bei dem erfindungsgemäßen Verfahren zur Aufreinigung von Acrylsäure herrschen in dem Aufschmelzer eine Temperatur im Bereich von 10 bis 50°C, vorzugsweise von 11 bis 30°C bei einem Druck von 1 bis 10 bar, vorzugsweise 3 bis 7 bar.

In den Führungen herrschen Temperatur- und Druckverhältnisse, die einen sicheren und störungsfreien Transport der (Meth)Acrylsäure und den diese ggf. begleitenden Stoffe in diesen Führungen erlauben.

Das erfindungsgemäße Verfahren erlaubt es, von einer verhältnismäßig verunreinigten (Meth)Acrylsäure auszugehen, wodurch der Voraufwand für eine Destillation der aus der Synthese stammenden (Meth)Acrylsäure geringer wird. Damit sinkt insbesondere die thermische Belastung der (Meth)Acrylsäure, die zu unerwünschten Polymerisationen führen kann.

Die erfindungsgemäßen Fasern, Formkörper, Filme, Schäume, superabsorbierenden Polymere oder Hygieneartikel basieren mindestens auf oder beinhalten mindestens die erfindungsgemäße (Meth)Acrylsäure.

Die erfindungsgemäßen (Meth)Acrylsäure wird erfindungsgemäße in oder zur Herstellung von Fasern, Formkörpern, Filmen, Schäumen, superabsorbierenden Polymeren oder Hygieneartikeln verwendet.

Die erfindungsgemäße Vorrichtung oder die erfindungsgemäße Aufreinigungsvorrichtung oder des erfindungsgemäßen Verfahrens wird erfindungsgemäß zur Herstellung von Acrylsäure verwendet, die eine Reinheit von mehr als 90 Gew.-%, bevorzugt mehr als 95 Gew.-% und besonders bevorzugt mehr als 99,5 Gew.-%, jeweils bezogen auf die (Meth)Acrylsäure mit Verunreinigungen, aufweist.

Die erfindungsgemäße Aufreinigungsvorrichtung oder das erfindungsgemäße Verfahren wird vorzugsweise zur Herstellung von Methacrylsäure verwendet, die eine Reinheit von mehr als 90 Gew.-%, bevorzugt mehr als 95 Gew.-% und besonders bevorzugt mehr als 99,5 Gew.-%, jeweils bezogen auf die (Meth)Acrylsäure mit Verunreinigungen, aufweist.

In einer speziellen Ausgestaltung des erfindungsgemäßen Verfahren zur Aufreinigung von (Meth)Acrylsäure weist das Verfahren zwei Verfahrensschritte auf:
γ1) Kristallisation von (Meth)Acrylsäure aus einem verunreinigten Roh-(Meth)Acrylsäurestrom aus einem Verfahren zur Herstellung von (Meth)Acrylsäure;
γ2) Abtrennen der (Meth)Acrylsäurekristalle aus der Mutterlauge mittels einer Waschkolonne, die Mutterlauge aus Schritt γ2 zumindest teilweise in den Schritt γ1) zurückführt, wobei der Roh-(Meth)Acrylsäurestrom vorzugsweise eine Reinheit von <99,5 Gew.-% an (Meth)Acrylsäure aufweist,

In Frage kommt für die Erfindung auch und in erster Linie eine mechanisch betriebene Waschkolonne (Diss. Poschmann, S.18).

Bei einer mechanischen Waschkolonne - beispielhaft wird auf die EP 0 193 226 B und NL 1007687 A verwiesen - wird ein dichtes Kristallbett innerhalb der Kolonne mittels eines für die Schmelze durchlässigen Kolbens erzeugt. Der Kolben kann sich am oberen oder unteren Ende der Kolonne befinden; im ersten Fall erfolgt der Zulauf der Suspension im oberen Bereich der Kolonne, im zweiten Fall im mittleren oder unteren Bereich. Der Kolben ist für die Schmelze durchlässig, so dass beim Komprimieren Schmelze an der Rückseite des Kolbens austritt und dort abgezogen wird. Analog wie eine hydraulische Waschkolonne enthält auch die mechanische Waschkolonne eine Vorrichtung zum Abkratzen, beispielsweise ein bewegtes, vorzugsweise rotierendes Kratzorgan, um Kristalle aus dem Kristallbett abzukratzen und mit der Waschschmelze in eine Suspension zu überführen. Die Waschschmelze bewegt sich im Gegenstrom zum Kristallbett. Von der dem Kolben gegenüberliegenden Seite der Waschkolonne wird Suspension abgezogen, und nach dem Aufschmelzen kann ein Teil der Schmelze als Waschschmelze zurückgeführt und der andere Teil als Reinstprodukt aus dem Kreislauf abgezogen werden.

Ausführungsformen zur Suspensionskristallisation mit nachgeschalteter Wäsche der Kristalle in einer hydraulischen oder mechanischen Waschkolonne sind dem Buch "Melt Crystallization Technology" von G.F. Arkenbout, Technomic Publishing Co. Inc., Lancaster-Basel (1995), S. 265-288 sowie dem sich auf die Niro-Gefrierkonzentration zur Abwasservorkonzentrierung richtenden Artikel in Chemie Ingenieur Technik (72) (10/2000), 1231-1233 zu entnehmen.

Als Waschflüssigkeit kann je nach Einsatzzweck eine dem Fachmann geläufige Waschflüssigkeit benutzt werden (bei wässrigen Lösungen z.B. Wasser). Ganz besonders bevorzugt dienen zum Waschen der kristallisierten (Meth)Acrylsäure wie schon angedeutet eine Teilmenge der aufgeschmolzenen Kristalle derselben. Durch diese Maßnahme wird zum einen gewährleistet, dass zur Produktion hochreiner Produkte kein weiterer Stoff in das System eingeführt werden muß, zum anderen dienen die aufgeschmolzenen Kristalle aber auch zum Zurückdrängen der Mutterlaugenfront in der Waschkolonnen und üben gleichzeitig auf die Kristalle einen reinigenden Effekt analog dem Schwitzen aus. Ein Produktverlust findet dabei nicht statt, da die Waschflüssigkeit auf den zu waschenden Kristallen wiederum auskristallisiert und sich so im Produkt wiederfindet (Z. B. Prospekt der Firma Niro Process Technology B.V., Crystallization and wash column separation set new standards in purity of chemical compounds).

In einer Ausgestaltung des erfindungsgemäßen Verfahrens wird die abgetrennte, insbesondere kristalline und/oder geschmolzene, (Meth)Acrylsäure in einem separaten Reinigungsverfahren gereinigt. Beispielsweise ist es möglich, die Mutterlauge aus Schritt b) vor dem Zurückführen in den Schritt a) mindestens ein Mal mit einem weiteren Reinigungsverfahren zu behandeln. Derartige Verfahren sind dem Fachmann hinlänglich bekannt. Vorzugsweise kommen als solche Verfahren im einzelnen folgende zur Anwendung:
1.) Einfache Destillation

Auftrennung in Leichtsieder (Essigsäure, Wasser usw.), Mittelsieder (Meth(Acrylsäure)) und Schwersieder (MSA, PTA usw.). Die Reinigung verunreinigter (Meth)Acrylsäure (insbes. von Wasser und Essigsäure) wird in einem Großteil der Fälle mittels Azeotroprektifikation vorgenommen. Zur Anwendung kommen beispielsweise Schleppmittel wie Toluol oder MIBK (EP 0 695 736 B1).
2.) Extraktion von Acrylsäure

Mit n-Butanol kann die (Meth)Acrylsäure extraktiv gewonnen werden. Übrig bleibt eine wässrige Phase, in der die Nebenkomponenten gelöst sind. Die Extraktion von (Meth)Acrylsäure aus verunreinigten Lösungen ist Stand der Technik wie die Destillation. Extrahiert werden kann (Meth)Acrylsäure beispielsweise auch aus wässrigen Lösungen mit flüssigen Ionenaustauschern, Mischungen aus tri-n-Alkylaminen und aliphatischen Alkoholen oder n-Butanol (Vogel et al.: Chem.Eng.Technol.23 (2000)1, pp. 70 - 74; Tamada et al.: in Solvent Extraction 1990, Ed.: T. Sekine, Elsevier Science Publishers B.V., pp. 1791 - 1796; JP 57 095 938; WO 98/40342; Informationsbroschüre der Firma SULZER Chemtech zur fraktionierten Extraktion von (METH)ACRYLSÄURE mit n-Butanol).
3.) Entwässerung von (Meth)Acrylsäure durch Pervaporation DE 4401405 A1

Vorteilhafterweise kann man die Mutterlauge aus Schritt b) vor dem Zurückführen in den Schritt a) mindestens ein Mal mit einem Verfahren aufweisend die Schritte a) und b) behandeln und den jeweils reineren abgezweigten Teilstrom in Schritt a) des Ausgangs- oder eines Vorgängerverfahrens zurückführen, um dadurch mit minimalisiertem Ausbeuteverlust ein Maximum an Reinheit zu erzeugen.

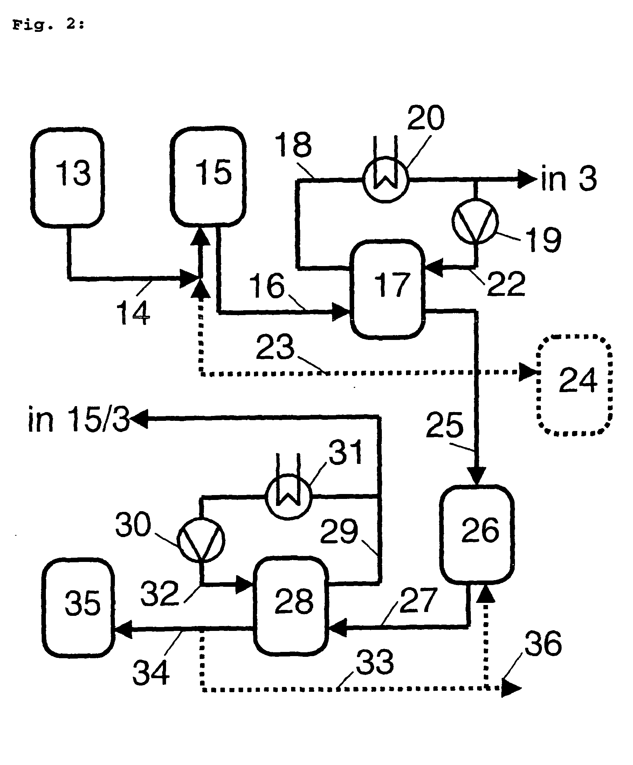

Fig. 2 erläutert diesen Sachverhalt. Die Mutterlauge der Kristalltrennung in der Waschkolonne kann ebenfalls in einem nächsten Kristallisationsgefäß behandelt werden. Die entstehende Suspension kann dann wiederum in einer Waschkolonne wie gehabt aufgearbeitet werden. Die jetzt entstandene Mutterlauge kann in folgenden Stufen analog behandelt werden. Man hat dabei die Wahl, die bei dieser Verfahrensweise anfallenden jeweils reineren Teilströme in die Kristallisation des ersten Reinigungsverfahrens zurückzuführen oder aber in die Kristallisation eines Vorgängerverfahrens. Derart kann mit relativ geringem apparativen Aufwand ein Maximum an Reinheit bei einem Minimum an Abfall realisiert werden.

Die erfindungsgemäßen Verfahren können kontinuierlich und diskontinuierlich betrieben werden, wobei der kontinuierliche Betrieb bevorzugt ist, da ein solcher Betrieb besonders wirtschaftlich ist.

In einer weiteren Ausgestaltung beschäftigt sich die Erfindung mit der Verwendung der erfindungsgemäß hergestellten (Meth)Acrylsäure in einem Verfahren zur Herstellung von Polymeren, vorzugsweise Superabsorbern, Detergentien oder Spezialpolymere für die Bereiche: Abwasserbehandlung, Dispersionsfarben, Kosmetika, Textilien, Lederveredlung, Papierherstellung.

Zudem betrifft die Erfindung Fasern, Formkörper, Filme, Schäume, superabsorbierenden Polymere oder Hygieneartikel, mindestens basierend auf oder beinhaltend die vorstehend beschriebene erfindungsgemäße (Meth)Acrylsäure.

Weiterhin betrifft die Erfindung die Verwendung der vorstehend beschriebene erfindungsgemäßen (Meth)Acrylsäure nach Anspruch 11 in oder zur Herstellung von Fasern, Formkörpern, Filmen, Schäumen, superabsorbierenden Polymeren oder Hygieneartikeln.

Vorteilhafterweise wird die erfindungsgemäße (Meth)Acrylsäure zur Herstellung von Polymeren, vorzugsweise Superabsorbern, Detergentien oder Spezialpolymeren für die Bereiche wie Abwasserbehandlung, Dispersionsfarben, Kosmetika, Textilien, Lederveredlung, Papierherstellung verwendet.

Unter superabsorbierenden Polymeren werden Polymere verstanden, die ein mehrfaches ihres Eigengewicht an Wasser oder wässrigen Flüssigkeiten aufnehmen. Vorzugsweise basieren superabsorbierende Polymere zu mehr als der Hälfte auf Acrylssäure als Monomer. Weitere Einzelheiten zu superabsorbierenden Polymeren, ihrer Herstellung und Verwendung in Hygieneartikeln sind "Modern Superabsorbent Polymer Technology", Fredric L. Buchholz, Andrew T. Graham, Wiley-VCH, 1998 zu entnehmen, auf dessen Inhalt als Teil dieser Offenbarung Bezug genommen wird.

Weiterhin stellen die sich aus den vorstehend mit Ziffern gekennzeichneten Merkmalen ergebenden Kombinationen von Merkmalen jeweils einzelne Ausführungsformen der vorliegenden Erfindung dar.

Weitere Einzelheiten und vorteilhafte Weiterbildungen werden anhand der folgenden Zeichnung, die die Erfindung exemplarisch veranschaulichen soll, näher erläutert.

Es zeigen schematisch:
- Fig. 1: ein weiteres erfindungsgemäßes Verfahrensschema nach Fig. 1;
- Fig. 2: ein erfindungsgemäßes Verfahrensschema mit zwei Verfahrensstufen in einer Reihenschaltung;
- Fig. 3: ein erfindungsgemäßes Verfahrensschema mit zwei Verfahrensstufen in Verschachtelung;
- Fig. 4: ein erfindungsgemäßes Verfahrensschema mit einer Verfahrensstufe; und
- Fig. 5: ein erfindungsgemäßes Verfahrensschema mit zwei Verfahrensstufen.

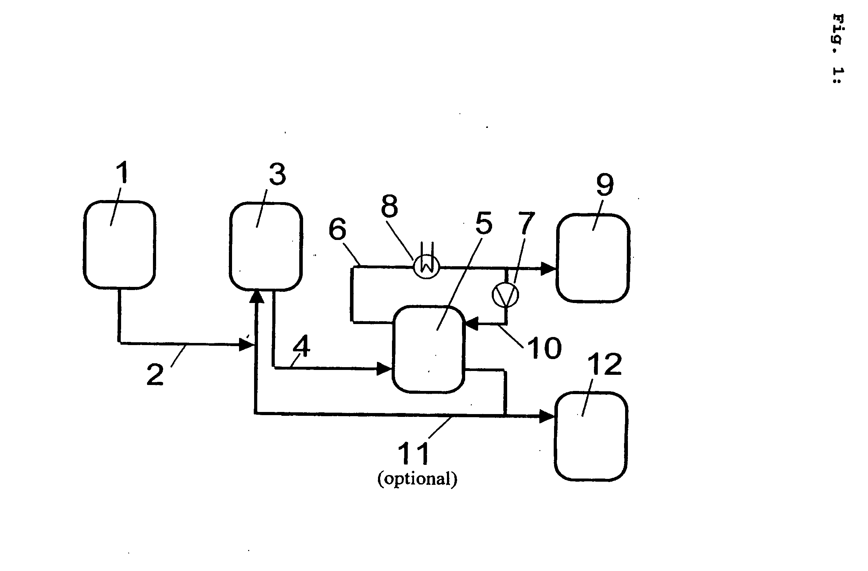

Die Figur 1 zeigt ein Verfahrensschema, bei welchem das Verfahren (in einer Stufe) zusammenfassend beschrieben wird:

### Stufe 1:

1. Vorratsbehälter
2. Zulauf 1 in 3
3. Suspensionserzeuger (z.B. Kühlscheibenkristallisator, Kratzkühler)
4. Zulauf 3 in 5
5. Waschkolonne, hydraulisch oder mechanisch
6. Produktkreislauf
7. Produktkreislaufpumpe
8. Produktkreislaufwärmetauscher
9. Produktbehälter
10. Produktrückführung zur Gegenstromwäsche in der Waschkolonne 5
11. Mutterlaugenrückführung (optional)
12. Mutterlaugenbehälter (Abstoss)

Die Waschkolonne 5, der Suspensionserzeuger 3, der Produktkreislaufwärmetauscher 8, der Zulauf 4, der Produktkreislauf 6 und die Produktrückführung 10 entsprechen dem Trennbereich, dem Kristallisationsbereich, dem Aufschmelzer, der ersten Führung, der zweiten Führung, der vierten beziehungsweise, wenn in der Waschkolonne Kristallbildung stattfindet, der dritten Führung.

Aus dem Vorratsbehälter 1 wird die zu trennende Schmelze im flüssigen Zustand, also knapp über Gleichgewicht temperiert über den Zulauf 2 in den Suspensionserzeuger 3 geführt. Durch Kühlung unter die Gleichgewichtstemperatur der Schmelze entstehen im Suspensionserzeuger 3 kontinuierlich Kristalle mit einer Suspensionsdichte zwischen 5 und 50% (vorzugsweise 20 bis 30%). Die Suspension wird kontinuierlich über den Zulauf 4 in die Waschkolonne 5 geführt und dort über bewegte oder feststehende Filter (hydraulisch oder mechanisch, s.o.) in eine flüssige und eine feste Phase getrennt. Das Filtrat verläßt die Waschkolonne 5 und wird dem Mutterlaugenbehälter 12 kontinuierlich zugeführt. Zur Ausbeutesteigerung ist es optional möglich, zumindest einen Teil des Filtrats auch über die Mutterlaugenrückführung 11 in den Suspensionserzeuger 3 zurückzuführen.

Die Kristalle in der Waschkolonne 5 werden zu einem Kristallbett verdichtet und je nach Typ der Waschkolonne oben oder unten mit umlaufenden Messern abgeschabt. Die abgeschabten Produktkristalle werden als Suspension im Produktkreislauf 6 mit der Kreislaufpumpe 7 umgepumpt und schmelzen durch das Einbringen der Schmelzenthalpie mit dem Wärmetauscher 8 auf.

Ein Teil wird als Waschflüssigkeit zur Gegenstromwäsche in die Waschkolonne durch die Produktrückführung 10 zurückgeführt. Der andere Teil verläßt die Anlage als Produkt in den Behälter 9.

Ist bei dieser einstufigen Fahrweise ein Minimum an Produktverlust durch den Abstoß nicht zu erreichen, kann die Mutterlauge aus der ersten Kristallisationsstufe optional in einer oder mehreren weiteren Kristallisationsstufen oder mit anderen Reinigungsmittel aufgearbeitet werden.

Figur 2 zeigt exemplarisch die Durchführung in mehreren Kristallisationsstufen

### Stufe 2/3 etc.:

13. Mutterlaugenbehälter der 1. Stufe (=12)
14. Zulauf aus 13 in 15
15. Suspensionserzeuger Stufe 2 (z.B. Kühlscheibenkristallisator, Kratzkühler)
16. Zulauf aus 15 in 17
17. Waschkolonne, hydraulisch oder mechanisch
18. Produktkreislauf der 2. Stufe
19. Produktkreislaufpumpe
20. Produktkreislaufwärmetauscher
21.
22. Produktrückführung in 17
23. Mutterlaugenrückführung in den Kristaller der 2. Stufe (optional)
24. Mutterlaugenbehälter der 2. Stufe (Abstoss)
25. Mutterlaugenzuführung aus der 2. Stufe in 26
26. Suspensionserzeuger Stufe 3 (z.B. Kühlscheibenkristallisator, Kratzkühler
27. Zuführung des Produktes aus 26 in 28
28. Waschkolonne, hydraulisch oder mechanisch
29. Produktkreislauf der 3. Stufe
30. Produktkreislaufpumpe
31. Produktkreislaufwärmetauscher
32. Produktrückführung in 28
33. Mutterlaugenrückführung in den Kristaller der 3. Stufe (optional)
34. Zulauf aus 28 in 35
35. Mutterlaugenbehälter (Abstoß) der 3. Stufe
36. ggf. Zuführung zu weiteren Reinigungsstufen

Der Suspensionserzeuger 15, die Waschkolonne 17, der Produktkreislaufwärmetauscher 20, der Zulauf 16, der Produktkreislauf 18, Produktrückführung 22, die Mutterlaugenrückführung 23 entsprechen jeweils bei der ersten Vorrichtungseinheit dem Kristallisationsbereich, dem Trennbereich und dem Aufschmelzer, der ersten Führung, der zweiten Führung, der dritten bzw. vierten Führung, der zweiten Rückführung.

Der Suspensionserzeuger 26, die Waschkolonne 28, und der Produktkreislaufwärmetauscher 31 der Produktkreislauf 32, die Produktrückführung 29, die Mutterlaugenrückführung 33 entsprechen jeweils bei der zweiten Vorrichtungseinheit dem Kristallisationsbereich, dem Trennbereich, dem Aufschmelzer, der zweiten Führung, der dritten bzw. vierten Führung, der zweiten Rückführung.

Die Mutterlaugenzuführung 25 entspricht einer Verbindungsleitung.

Die Mutterlauge (12/13) aus der 1. Stufe wird teilweise oder vollständig in den Suspensionserzeuger der 2. Stufe 15 geführt. Durch Kühlung unter die Gleichgewichtstemperatur der Schmelze entstehen im Suspensionserzeuger 15 kontinuierlich Kristalle mit einer Suspensionsdichte zwischen 5 und 50% (vorzugsweise 20 bis 30%). Die Suspension wird kontinuierlich über den Zulauf 16 in die Waschkolonne der 2. Stufe (17) geführt und dort über bewegte oder feststehende Filter (hydraulisch oder mechanisch, s.o.) in eine flüssige und eine feste Phase getrennt. Zu einem Kristallbett verdichtet werden die Kristalle je nach Typ der Waschkolonne 17 oben oder unten mit umlaufenden Messern abgeschabt. Die abgeschabten Produktkristalle werden optional (wie in der 1. Stufe) als Suspension im Produktkreislauf 18 mit der Kreislaufpumpe 19 umgepumpt und schmelzen durch das Einbringen der Schmelzenthalpie mit dem Wärmetauscher 20 auf. Ein Teil kann als Waschflüssigkeit zur Gegenstromwäsche in die Waschkolonne zurückgeführt werden (22). Der andere Teil kann als aufgeschmolzenes Produkt in den Suspensionserzeuger der ersten Stufe (3) zurückgeführt werden. Das Filtrat verläßt die Waschkolonne 17 und wird dem Mutterlaugenbehälter 24 kontinuierlich zugeführt. Zur weiteren Ausbeutesteigerung ist es optional möglich, zumindest einen Teil des Filtrats auch über die Mutterlaugenrückführung 23 in den Suspensionserzeuger 15 zurückzuführen und/oder in einer 3. Stufe weiter aufzuarbeiten. Dazu wird die Mutterlauge über 25 in einen weiteren Suspensionserzeuger 26 geleitet. Die wie oben gewonnene Suspension wird über 27 in die Waschkolonne 28 geleitet, dort zu einem Kristallbett verdichtet und je nach Typ der Waschkolonne 28 oben oder unten mit umlaufenden Messern abgeschabt. Die abgeschabten Produktkristalle werden optional (wie in der 1. Stufe) als Suspension im Produktkreislauf 29 mit der Kreislaufpumpe 30 umgepumpt und schmelzen durch das Einbringen der Schmelzenthalpie mit dem Wärmetauscher 31 auf.

Ein Teil kann als Waschflüssigkeit zur Gegenstromwäsche in die Waschkolonne zurückgeführt werden (32). Der andere Teil kann als aufgeschmolzenes Produkt in den Suspensionserzeuger der ersten Stufe 3 oder der 2. Stufe 15 zurückgeführt werden.

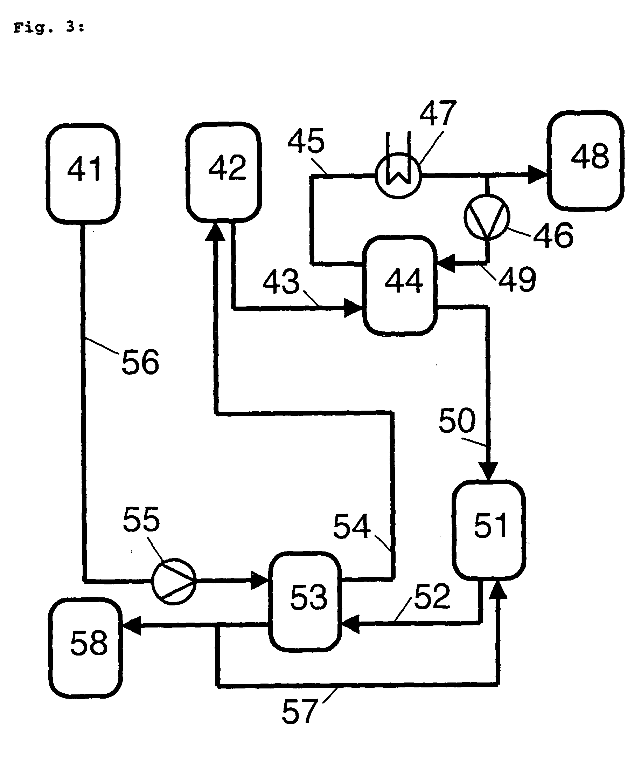

Figur 3 zeigt eine weitere bevorzugte Verschaltung des erfindungsgemäßen Aufarbeitungsverfahrens.
41. Vorratsbehälter
42. Suspensionserzeuger (z.B. Kühlscheibenkristallisator, Kratzkühler)
43. Zulauf 42 in 44
44. Waschkolonne, hydraulisch oder mechanisch
45. Produktkreislauf
46. Produktkreislaufpumpe
47. Produktkreislaufwärmetauscher
48. Produktbehälter
49. Produktrückführung zur Gegenstromwäsche in der Waschkolonne 44
50. Zulauf der 2. Stufe = Mutterlauge aus Stufe 1 (aus der Waschkolonne 44)
51. Suspensionserzeuger Stufe 2 (z.B. Kühlscheibenkristallisator, Kratzkühler)
52. Zulauf 51 in 53
53. Waschkolonne, hydraulisch oder mechanisch
54. Produkt aus der 2. Stufe (Suspension, vermischt mit dem Original aus 41, der nicht in Stufe 1, sondern in Stufe 2 geführt wird), das in den Kratzkühler der 1. Stufe (42) geführt wird
55. Produktzuführungspumpe
56. Zuführung aus der ersten Stufe (41) direkt in den Kopf der Waschkolonne 53 der 2. Stufe
57. Mutterlaugenrückführung in den Kristaller der zweiten Stufe (51) (optional)
58. Mutterlaugenbehälter der zweiten Stufe (Abstoss)

Der Suspensionserzeuger 42, die Waschkolonne 44, der Produktkreislaufwärmetauscher 47, der Produktkreislauf 45, Produktrückführung 49, der Zulauf 43 entsprechen jeweils bei der ersten Vorrichtungseinheit dem Kristallisationsbereich, dem Trennbereich und dem Aufschmelzer, der zweiten Führung, der dritten bzw. vierten Führung, der ersten Führung.

Der Suspensionserzeuger 51, die Waschkolonne 53, der Zulauf 52, die Mutterlaugenrückführung 57 entsprechen jeweils bei der zweiten Vorrichtungseinheit dem Kristallisationsbereich, dem Trennbereich, der ersten Führung, der zweiten Rückführung.

Die Zuläufe 54, 50 entsprechen Verbindungsleitungen. Die hiermit beispielhaft dargestellte Verschachtelung erlaubt vorteilhafterweise, dass die zweite Vorrichtungseinheit keinen Aufschmelzer benötigt.

Eine besonders bevorzugte Variante einer zwei- oder mehrstufigen Ausführung führt die zu trennende Schmelze der ersten Stufe 41 über die Zuführung 56 und die Pumpe 55 in den Kopf der Waschkolonne der 2. Stufe 53 um dort die abgeschabten Produktkristalle der zweiten Stufe als Suspension 54 in den Suspensionserzeuger der 1. Stufe 42 zu führen. Diese Variante hat den energetischen Vorteil, auf ein Aufschmelzen in der zweiten Stufe verzichten zu können und die nun in der ersten Stufe vorhandenen Kristalle nicht noch einmal ausfrieren zu müssen.

Das Gegenstromwaschen in der Waschkolonne der 2. Stufe 53 wird hier durch die Schmelze aus 41 über Zulauf 56 der 1. Stufe erreicht, der im Vergleich zur Verunreinigungskonzentration in der 2. Stufe sehr rein ist und von daher ein ähnlich effektives Waschen wie ein Waschen mit Produkt ermöglicht.

Obgleich eine Kombination aus einem Kratzkühler und einer Waschkolonne zur Herstellung organischer Substanzen mit hoher Reinheit bekannt ist, war nicht vorhersehbar, dass dieses Verfahren auch zur Konzentrierung von (Meth)Acrylsäure mit Ausgangsreinheiten von <99,5 Gew.-% sehr gut geeignet ist. Im Hinblick auf die unbekannten Kristallisationseigenschaften von (Meth)Acrylsäure geringerer Reinheit war nicht zu erwarten, dass in einer Stufe eine Konzentrierung von über 14% realisiert werden kann. Überraschend war ferner, dass der Gehalt an Verunreinigungen, wie weitere organische Kohlenstoffverbindungen, durch das erfindungsgemäße Verfahren auf Werte unterhalb der Nachweisgrenze gesenkt werden kann. Dergestalt können gerade auch kritische Größen wie Fufural, Inhibitoren, Essigsäure oder Maleinsäure auf unkritische Werte abgereichert werden. Dies ist so dem Stand der Technik nicht zu entnehmen gewesen bzw. wird durch diesen auch nicht nahegelegt.

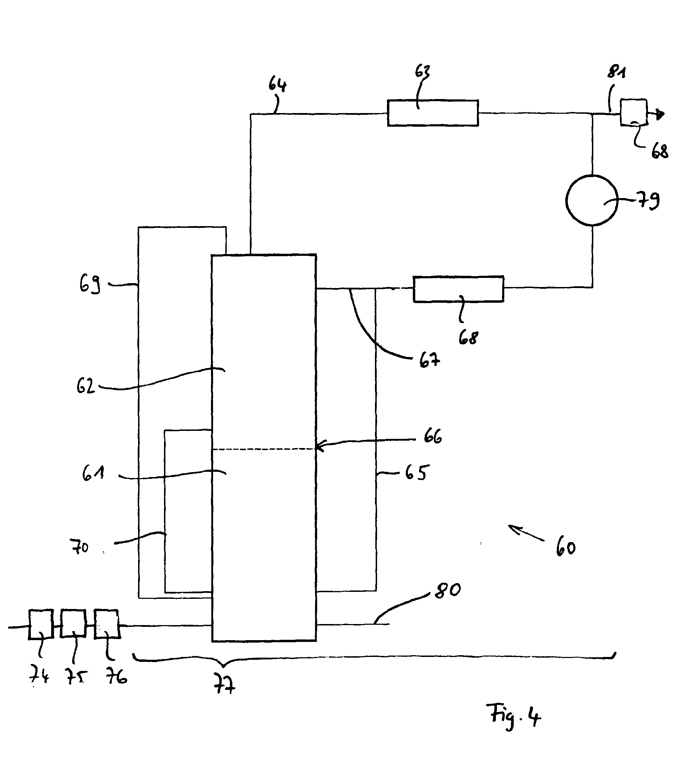

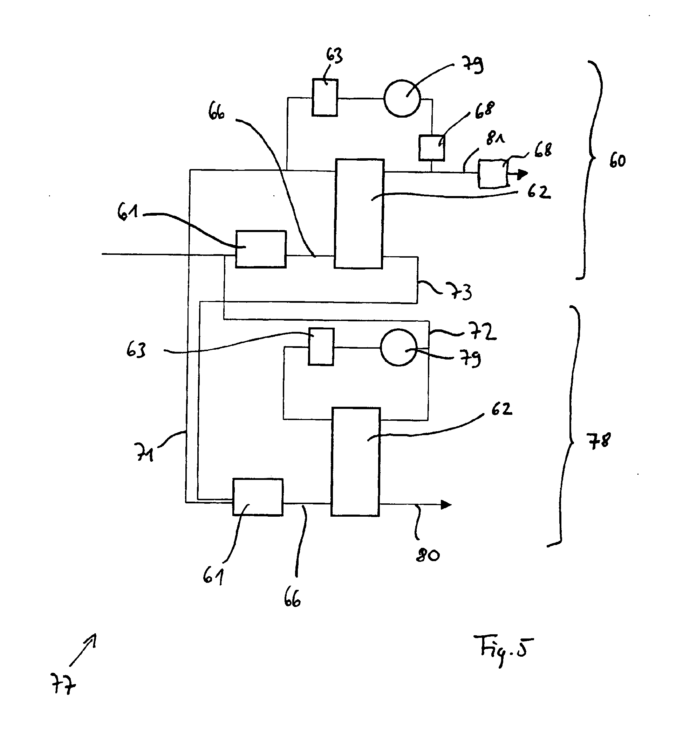

Die Fig. 4 zeigt ein erfindungsgemäßes Verfahrensschema mit einer Verfahrensstufe und die Fig. 5 zeigt ein erfindungsgemäßes Verfahrensschema mit zwei Verfahrensstufen.
60. Vorrichtungseinheit (1. Verfahrensstufe)
61. Kristallisationsbereich
62. Trennbereich
63. Aufschmelzer
64. zweite Führung
65. dritte Führung
66. erste Führung
67. vierte Führung
68. separate Reinigungsvorrichtung
69. erste Rückführung
70. zweite Rückführung
71. Verbindungsleitung
72. Verbindungsleitung
73. Verbindungsleitung
74. (Meth)ACrylsäure-Reaktor
75. Quenchtower
76. Destillationsvorrichtung
77. Aufreinigungsvorrichtung
78. Vorrichtungseinheit (2. Verfahrensstufe)
79. Pumpe
80. Restmutterlaugenablauf
81. Produktablauf

Fig. 4 zeigt eine erfindungsgemäße Vorrichtung zur Herstellung von (Meth)Acrylsäure mit einem (Meth)Acrylsäure-Reaktor 74, einem Quenchtower 75, einer Destillationsvorrichtung 76 und einer Aufreinigungsvorrichtung 77, wobei die Aufreinigungsvorrichtung 77 einen Kristallisationsbereich 61 und einen Trennbereich 62 enthält. Der Kristallisationsbereich 61 und der Trennbereich 62 liegen eng beieinander, vorzugsweise sind der Kristallisationsbereich 61 und der Trennbereich 62 nicht über Rohrleitungen, sondern direkt miteinander verbunden. Vorzugsweise findet in einem einzigen Gehäuse während des Waschens der Kristalle, insbesondere der Abtrennung der Kristalle von der Mutterlauge, auch ein Kristallwachstum statt. Der Kristallisationsbereich 61 und der Trennbereich 62 sind durch eine erste Führung 66, die durch das gemeinsame Gehäuse gebildet sein kann, miteinander verbunden. Der Trennbereich 62 ist über eine zweite Führung 74 mit dem Aufschmelzer 63 verbunden, der die im Abtrennbereich von der Mutterlauge abgetrennten (Meth)Acrylsäurekristalle aufschmilzt. Die aufgeschmolzene (Meth)Acrylsäure wird mit Hilfe einer Pumpe 79 entweder über eine vierte Führung 67 zum Trennbereich 62 oder über eine dritte Führung 65 zum Kristallisationsbereich 61 befördert. Vorteilhafterweise sorgt eine separate Reinigungsvorrichtung 68 für eine Steigerung der Reinheit der (Meth)Acrylsäure. Um die Kristallisation im Kristallisationsbereich 61 zu fördern werden mit Hilfe einer ersten Rückführung 69 im Trennbereich 62 abgetrennt (Meth)Acrylsäurekristalle als Impfkristalle in den Kristallisationsbereich 61 zugeführt. Die Ausbeute wird gesteigert, in dem die bei einer unvollständigen Abtrennung im Trennbereich 62 in der Mutterlauge verbliebene (Meth)Acrylsäure zurückgewonnen wird, indem die Mutterlauge aus dem Trennbereich 62 über eine zweite Rückführung 70 dem Kristallisationsbereich 61 zugeführt wird. Mit Hilfe eines Produktablaufes 81 wird die gereinigte (Meth)Acrylsäure dem Kreislauf entnommen. Mittels eines Restmutterlaugenablaufes 80 wird nicht mehr verwertbare Restmutterlauge entnommen.

Die Figur 5 zeigt eine Aufreinigungsvorrichtung 77 mit zwei Vorrichtungseinheiten 60, 78. Damit umfasst die Aufreinigungsvorrichtung 77 zwei Verfahrenstufen. Um die erzielbare Reinheit der (Meth)Acrylsäure sowie die Ausbeute zu steigern werden die beiden Vorrichtungseinheiten 60 mit Verbindungsleitungen 71, 72, 73 miteinander verbunden.

In Bezug auf die Mutterlauge, welche von der ersten Vorrichtungseinheit 60 über eine Verbindungsleitung 73 der zweiten Vorrichtungseinheit 78 zuführt wird, sind die beiden Vorrichtungseinheiten 60 und 78 in Reihe verschaltet.

Zur Förderung des Kristallwachstums in der zweiten Vorrichtungseinheit 78 werden abgetrennte (Meth)Acrylsäurekristalle aus dem Trennbereich 62 der ersten Vorrichtungseinheit 60 über eine Verbindungsleitung 71 dem Kristallisationsbereich 61 der zweiten Vorrichtungseinheit 78 zugeführt. Dieses ist insbesondere dann zweckmäßig, wenn die Konzentration der (Meth)Acrylsäure in der der zweiten Vorrichtungseinheit 78 zugeführten Mutterlauge schon nahe bei dem Konzentrationsgrenzwert für eine Auskristallisation von (Meth)Acrylsäure aus der Mutterlauge liegt. Durch diese Maßnahme wird die Ausbeute der Aufreinigungsvorrichtung 77 insgesamt gesteigert.

Um die Reinheit der aus der zweiten Verfahrenstufe mit der zweiten Vorrichtungseinheit 78 gewonnenen (Meth)Acrylsäure zu steigern, die aufgrund der niedrigeren (Meth)Acrylsäurekonzentration in der Mutterlauge, die der zweiten Vorrichtungseinheit 78 zugeführt wird, niedriger ausfallen kann, wird zu weiteren Aufreinigung die in der zweiten Verfahrenstufe gewonnene (Meth)Acrylsäure über eine Verbindungsleitung 72 dem Kristallisationsbereich 61 der ersten Vorrichtungseinheit 60 zugeführt. Durch diese Nachreinigung wird ein verbesserte Reinheit der gewonnen (Meth)Acrylsäure gewährleistet.

Im Bezug auf den Transport der Mutterlauge folgt die zweite Vorrichtungseinheit 78 der ersten Vorrichtungseinheit 60. In Bezug auf den Transport der (Meth)Acrylsäure folgt die erste Vorrichtungseinheit 60 der zweiten Vorrichtungseinheit 78. Damit sind die beiden Vorrichtungseinheiten 78 und 60 miteinander verschachtelt.

Die Erfindung wird nun anhand nicht limitierender Beispiele näher erläutert.

### BEISPIELE

### Ausführungsbeispiele Acrylsäure- und Methacrylsäure-Kristallisation

In einer Vorrichtung entsprechend der Figur 1 mit einem Kratzkristaller zur Suspensionserzeugung und einer mechanischen Waschkolonne mit unten angeordnetem Kolben und oben angeordnetem Abzug der gereinigten Schmelze wurde Acrylsäure mit folgender Zusammensetzung (Tabelle 1) in den Kratzkühler vorgelegt.

**Tabelle 1**

| Name | | Vorlage |
|---|---|---|
| Farbzahl | - | >700 |
| Wasser | % | 0,540 |
| Essigsäure | % | 0,061 |
| Furfural | % | 0,052 |
| Benzaldehyd | % | 0,141 |
| Propionsäure | % | 0,049 |
| Acrolein | % | 0,0007 |
| Protoanemonin | % | 0,039 |
| Acrylsäure | % | 93,9 |
| MEHQ | % | 0,081 |
| HQ | % | 0,101 |
| PZ | % | 0,161 |
| D-Acrylsäure | % | 2,3 |
| MSA | % | 1,52 |
| Sonstige | % | 1,06 |

Zur Erhöhung der Verunreinigungskonzentration im Kratzkühler wurde zu Beginn zusätzlich ca. 8% Wasser zudosiert. Als Feed wurde Acrylsäure einer in Tabelle 2 dargestellten Zusammensetzung verwendet.

**Tabelle 2**

| Name | | Edukt |
|---|---|---|
| Farbzahl | - | 3 |
| Wasser | % | 0,27 |
| Essigsäure | % | 0,026 |
| Furfural | % | 0,0119 |
| Benzaldehyd | % | 0,0023 |
| Propionsäure | % | 0,031 |
| Acrolein | % | |
| Protoanemonin | % | 0,0014 |
| Acrylsäure | % | 99,766 |
| MEHQ | % | 0,044 |
| HQ | % | <0,001 |
| PZ | % | <0,0001 |
| D-Acrylsäure | % | 0,09 |
| MSA | % | <0,005 |
| Sonstige | % | |

Der Kratzkühler wurde abgekühlt, wobei es im Kratzkühler bei ca. 5°C zur Ausbildung einer Kristallschicht kam, die durch die umlaufenden Schaber im Kratzkühler zur Bildung einer Suspension abgeschabt wurde.

Die abfiltrierte Mutterlauge wurde stets vollständig in den Kratzkühler zurückgeführt. Dadurch konzentrierten alle Nebenkomponenten im Kreislauf auf, so dass während der Versuche der Acrylsäuregehalt im Kratzkühler stetig abnahm. Dies wurde mit einer kontinuierlichen Absenkung der Kratzkühlertemperatur auf etwa 2°C kompensiert, um eine stete Kristallbildung wegen der Absenkung der Gleichgewichtstemperatur zu ermöglichen. Nach ca. 24 stündigem Betrieb wies die flüssige Phase im Kristaller (=Filtrat aus der Waschkolonne) die Zusammensetzung nach Tabelle 3 auf.

**Tabelle 3**

| Name | | Filtrat |
|---|---|---|
| Farbzahl | - | >700 |
| Wasser | % | 9,08 |
| Essigsäure | % | 0,178 |
| Furfural | % | 0,14 |
| Benzaldehyd | % | 0,18 |
| Acrolein | % | 0,115 |
| Propionsäure | % | 0,0018 |
| Protoanemonin | % | 0,06 |
| Acrylsäure | % | 85,8 |
| MEHQ | % | 0,219 |
| HQ | % | 0,115 |
| PZ | % | 0,167 |
| D-Acrylsäure | % | 0,3 |
| MSA | % | 2,1 |
| Sonstige | % | 1,56 |

Die aus dem Kristaller abgezogene Kristallsuspension wurde in der Waschkolonne zu einem kompakten Kristallbett verdichtet. An der Oberseite des Kristallbetts wurde dieses mittels eines rotierenden Schabers abgeschabt, als Kristallsuspension im Produktkreislauf umgepumpt und durch den Wärmetauscher aufgeschmolzen. Ein Teil wurde zur Gegenstromwäsche in das Kristallbett zurückgeführt. Der andere Teil wurde kontinuierlich als Produkt gewonnen. Exemplarisch für die erreichten Produktqualitäten ist in der Tabelle 4 die Produktanalyse derjenigen Acrylsäure genannt, die ca. 1 Stunde später als die Probe aus Tabelle 3 genommen wurde, also ca. 25 Stunden nach Versuchsbeginn.

**Tabelle 4**

| Name | | Produkt |
|---|---|---|
| Farbzahl | - | 8 |
| Wasser | % | 0,019 |
| Essigsäure | % | 0,011 |
| Furfural | % | <0,0001 |
| Benzaldehyd | % | <0,0001 |
| Propionsäure | % | 0,021 |
| Acrolein | % | <0,0001 |
| Protoanemonin | % | <0,0001 |
| Acrylsäure | % | 99,914 |
| MEHQ | % | 0,025* |
| HQ | % | <0,001 |
| PZ | % | <0,0001 |
| D-Acrylsäure | % | 0,01 |
| MSA | % | <0,005 |
| Sonstige | % | - |

| | | |
|---|---|---|
| *Produkt nachstabilisiert | | |

Wie aus Tabelle 4 ersichtlich, ermöglicht das erfindungsgemäße verfahren die Herstellung hochreiner Acrylsäure. Es wurden durchschnittlich 6 kg/h produziert.

Anstelle von Acrylsäure wurde in den Kratzkühler Methacrylsäure mit einer Zusammensetzung nach Tabelle 5 vorgelegt und zusätzlich ca. 2,5% Wasser zudosiert.

**Tabelle 5**

| Name | | Vorlage |
|---|---|---|
| Farbzahl | - | >700 |
| Wasser | % | 1,8 |
| Hydroxy-Isobuttersäure | % | 1,14 |
| Methacrylamid | % | 0,65 |
| Methylmethacrylat | % | 0,21 |
| Methacrylsäure | % | 95,0 |
| Essigsäure | % | 0,16 |
| Iso-Buttersäure | % | <0,01 |
| Acrylsäure | % | <0,01 |
| Propionsäure | % | 0,01 |
| MeHQ | % | 0,0281 |
| HQ | % | 0,0058 |

Methacrylsäure mit derselben Zusammensetzung nach Tabelle 5 wurde für die Versuche als Feed verwendet.

Der Kratzkühler wurde abgekühlt, wobei es im Kratzkühler bei ca. 9°C zur Ausbildung einer Kristallschicht kam, die durch die umlaufenden Schaber im Kratzkühler zur Bildung einer Suspension abgeschabt wurden.

Die abfiltrierte Mutterlauge wurde stets vollständig in den Kratzkühler zurückgeführt. Dadurch konzentrierten alle Nebenkomponenten im Kreislauf auf, so dass während der Versuche der Methacrylsäuregehalt im Kratzkühler stetig abnahm. Dies wurde mit einer kontinuierlichen Absenkung der Kratzkühlertemperatur auf etwa 6°C kompensiert, um eine stete Kristallbildung wegen der Absenkung der Gleichgewichtstemperatur zu ermöglichen. Nach ca. 4 stündigem Betrieb wies die flüssige Phase im Kristaller (=Filtrat aus der Waschkolonne) die Zusammensetzung nach Tabelle 6 auf.

**Tabelle 6**

| Name | | Filtrat |
|---|---|---|
| Farbzahl | - | >700 |
| Wasser | % | 5,5 |
| Hydroxy-Isobuttersäure | % | 2,2 |
| Methacrylamid | % | 1,00 |
| Methylmethacrylat | % | 0,32 |
| Methacrylsäure | % | 89,2 |
| Essigsäure | % | 0,3 |
| Iso-Buttersäure | % | <0,01 |
| Acrylsäure | % | <0,01 |
| Propionsäure | % | 0,01 |
| MeHQ | % | 0,0488 |
| HQ | % | 0,0053 |

Die aus dem Kristaller abgezogene Kristallsuspension wurde in der Waschkolonne zu einem kompakten Kristallbett verdichtet. An der Oberseite des Kristallbetts wurde dieses mittels eines rotierenden Schabers abgeschabt, als Kristallsuspension im Produktkreislauf umgepumpt und durch den Wärmetauscher aufgeschmolzen. Ein Teil wurde zur Gegenstromwäsche in das Kristallbett zurückgeführt. Der andere Teil wurde kontinuierlich als Produkt gewonnen. Exemplarisch für die erreichten Produktqualitäten ist in der Tabelle 7 die Produktanalyse derjenigen Methacrylsäure genannt, die ca. 1 Stunde später als die Probe aus Tabelle 6 genommen wurde, also ca. 5 Stunden nach Versuchsbeginn.

**Tabelle 7**

| Name | | Produkt |
|---|---|---|
| Farbzahl | - | 53 |
| Wasser | % | 0,12 |
| Hydroxy-Isobuttersäure | % | 0,03 |
| Methacrylamid | % | 0,07 |
| Methylmethacrylat | % | 0,01 |
| Methacrylsäure | % | 99,8 |
| Essigsäure | % | 0,02 |
| Iso-Buttersäure | % | <0,01 |
| Acrylsäure | % | <0,01 |
| Propionsäure | % | <0,01 |
| MeHQ | % | 0,0065 |
| HQ | % | 0,0074 |

Wie aus Tabelle 4 ersichtlich, ermöglicht das erfindungsgemäße Verfahren die Herstellung hochreiner Methacrylsäure. Es wurden durchschnittlich 6,5 kg/h produziert.

Die Konzentrationsangaben wurden per GC ermittelt. Die Farbzahl nach der Methode DIN-ISO 6271. Wasser wurde nach ASTM D 1364 und die Inhibitoren (MEHQ) nach ASTM D 3125 bestimmt.

## Patentansprüche

1. Verfahren zur Aufreinigung von Acrylsäure mit einer Verfahrensstufe, welche folgende Verfahrensschritte umfasst:
a) Acrylsäure wird aus einer Mutterlauge auskristallisiert;
b) kristallisierte Acrylsäure wird von der Mutterlauge abgetrennt;
c) zumindest ein Teil der abgetrennten Acrylsäurekristalle wird aufgeschmolzen; und
d) der aufgeschmolzene Teil wird dem Schritt a) oder dem Schritt b) zumindest teilweise zurückgeführt.

2. Verfahren zur Aufreinigung von Methacrylsäure mit einer Verfahrensstufe, welche folgende Verfahrensschritte umfasst:
a) Methacrylsäure wird aus einer Mutterlauge, auskristallisiert;
b) kristallisierte Methacrylsäure wird von der Mutterlauge, abgetrennt;
c) zumindest ein Teil der abgetrennten Methacrylsäurekristalle wird aufgeschmolzen; und
d) der aufgeschmolzene Teil wird dem Schritt a) oder dem Schritt b) zumindest teilweise zurückgeführt
wobei die im Verfahrensschritt a) eingesetzte Methacrylsäure ein Roh-Methacrylsäurestrom ist, der erhalten wurde durch Destillation einer wässrigen Methacrylsäurelösung in einer Destillationsvorrichtung, wobei die wässrige Methacrylsäurelösung durch Kondensation des Gemisches einer katalytischen Gasphasenoxidation von Isobuten, Isobutan, tert-Butanol, iso-Butyraldehyd, Methacrolein oder Meth-tert-Butylether mit Sauerstoff in einem Quenchtower erhalten wurde.

3. Verfahren nach Anspruch 1 oder 2, **dadurch gekennzeichnet, dass** die kristallisierte (Meth)Acrylsäure im Gegenstrom von der zurückgeführten (Meth)Acrylsäure gewaschen wird.

4. Verfahren nach einem der vorherigen Ansprüche, **dadurch gekennzeichnet, dass** abgetrennte (Meth)Acrylsäure in einem separaten Reinigungsverfahren gereinigt wird.

5. Verfahren nach einem der vorherigen Ansprüche, **dadurch gekennzeichnet, dass** die abgetrennte, kristallisierte (Meth)Acrylsäure aus Schritt b) zumindest teilweise in den Schritt a) zugeführt wird.

6. Verfahren nach einem der vorherigen Ansprüche, **dadurch gekennzeichnet, dass** die nach dem Schritt b) abgetrennte Mutterlauge zumindest teilweise in den Schritt a) zurückgeführt wird.

7. Verfahren nach einem der vorherigen Ansprüche, **gekennzeichnet durch** mindestens zwei Verfahrensstufen, die jeweils die Schritte a) bis d) aufweisen, wobei mindestens eines der folgenden Merkmale (α1) bis(α4) erfüllt ist:
(α1) abgetrennte (Meth)Acrylsäure aus einer ersten Verfahrensstufe wird zumindest teilweise einer zweiten Verfahrensstufe zugeführt;
(α2) abgetrennte (Meth)Acrylsäure aus einer zweiten Verfahrensstufe wird zumindest teilweise einer ersten Verfahrensstufe zugeführt;
(α3) Mutterlauge einer ersten Verfahrensstufe wird zumindest teilweise einer zweiten Verfahrensstufe zugeführt;
(α4) Mutterlauge einer zweiten Verfahrensstufe wird zumindest teilweise einer ersten Verfahrensstufe zugeführt.

8. Verfahren nach einem der vorherigen Ansprüche, **gekennzeichnet durch**
(γ1) Kristallisation von (Meth)Acrylsäure aus einem verunreinigten Roh-(Meth)Acrylsäurestrom aus einem Verfahren zur Herstellung von (Meth)Acrylsäure,
(γ2) Abtrennen der (Meth)Acrylsäurekristalle aus der Mutterlauge mittels einer Waschkolonne, wobei man die Mutterlauge aus Schritt zumindest teilweise in den Schritt (γ1) zu rückführt, wobei der Roh-(Meth)Acrylsäurestrom eine Reinheit von <99,5 Gew.-% an (Meth)Acrylsäure aufweist.

9. Vorrichtung zur Herstellung von (Meth)Acrylsäure umfassend als fluidleitend miteinander verbundene Komponenten einen (Meth)Acrylsäure-Reaktor, einen Quenchtower, eine Destillationsvorrichtung und eine Aufreinigungsvorrichtung, die eine Vorrichtungseinheit aufweist, welche die Merkmale(δ1) bis (δ4) umfasst
(δ1) die Vorrichtungseinheit weist einen Kristallisationsbereich, einen Trennbereich, einen Aufschmelzer, und mindestens drei Führungen auf;
(δ2) der Kristallisationsbereich ist mit dem Trennbereich über eine erste Führung verbunden;
(δ3) der Trennbereich ist mit dem Aufschmelzer über eine zweite Führung verbunden;
(δ4) der Aufschmelzer ist mit dem Kristallisationsbereich über eine dritte Führung verbunden.

10. Vorrichtung nach Anspruch 9, wobei die Vorrichtungseinheit eine separate Reinigungsvorrichtung aufweist.

11. Vorrichtung nach einem der Ansprüche 9 oder 10, wobei der Trennbereich mit dem Kristallisationsbereich durch eine erste Rückführung für abgetrennte (Meth)Acrylsäure verbunden ist.

12. Vorrichtung nach einem der Ansprüche 9 bis 11, wobei der Trennbereich mit dem Kristallisationsbereich durch eine zweite Rückführung für abgetrennte Mutterlauge verbunden ist.

13. Vorrichtung nach einem der Ansprüche 9 bis 12, **gekennzeichnet durch** mindestens zwei Vorrichtungseinheiten gemäss den Merkmalen (δ1) bis (δ4), die **durch** mindestens eine Verbindungsleitung verbunden sind, wobei die Verbindungsleitung eine Zuleitung oder eine Rückleitung ist, und wobei mindestens eines der folgenden Merkmale (ε1) bis (ε4) erfüllt ist:
(ε1) der Trennbereich einer ersten Vorrichtungseinheit ist über die Verbindungsleitung mit dem Kristallisationsbereich einer zweiten Vorrichtungseinheit verbunden;
(ε2) der Aufschmelzer einer ersten Vorrichtungseinheit ist über die Verbindungsleitung mit dem Kristallisationsbereich einer zweiten Vorrichtungseinheit verbunden;
(ε3) der Trennbereich einer zweiten Vorrichtungseinheit ist über die Verbindungsleitung mit dem Kristallisationsbereich einer ersten Vorrichtungseinheit verbunden;
(ε4) der Aufschmelzer einer zweit Vorrichtungseinheit ist über die Verbindungsleitung mit dem Kristallisationsbereich einer ersten Vorrichtungseinheit verbunden.

14. Vorrichtung nach einem des Ansprüche 9 bis 13, wobei der Aufschmelzer mit dem Trennbereich über eine vierte Führung verbunden ist.

15. Verwendung einer Vorrichtung, wie in einem der Ansprüche 9 bis 14 definiert, oder einer Aufreinigungsvorrichtung, wie in einem der Ansprüche 9 bis 14 definiert, oder eines Verfahrens, wie in einem der Ansprüche 1 bis 8 definiert, zur Herstellung von (Meth)Acrylsäure, die eine Reinheit von mehr als 90Gew.-%, bevorzugt mehr als 95 Gew.-% und besonders bevorzugt mehr als 99,5 Gew.-%, jeweils bezogen auf die (Meth)Acrylsäure mit Verunreinigungen, aufweist.

## Claims

1. Process for purifying acrylic acid, having a process stage which comprises the following process steps:
a) acrylic acid is crystallized out of a mother liquor;
b) crystallized acrylic acid is removed from the mother liquor;
c) at least some of the acrylic acid crystals removed are melted; and
d) the molten portion is recycled at least partly to step a) or to step b).

2. Process for purifying methacrylic acid, having a process stage which comprises the following process steps:
a) methacrylic acid is crystallized out of a mother liquor;
b) crystallized methacrylic acid is removed from the mother liquor;
c) at least some of the methacrylic acid crystals removed are melted; and
d) the molten portion is recycled at least partly to step a) or to step b),
the methacrylic acid used in process step a) being a crude methacrylic acid stream which has been obtained by distilling an aqueous methacrylic acid solution in a distillation apparatus, said aqueous methacrylic acid solution having been obtained by condensing the mixture from a catalytic gas phase oxidation of isobutene, isobutane, tert-butanol, isobutyraldehyde, methacrolein or methyl tert-butyl ether with oxygen in a quench tower.

3. Process according to Claim 1 or 2, **characterized in that** the crystallized (meth)acrylic acid is washed in countercurrent by the recycled (meth)acrylic acid.

4. Process according to any of the preceding claims, **characterized in that** removed (meth)acrylic acid is purified in a separate purification process.

5. Process according to any of the preceding claims, **characterized in that** the removed, crystallized (meth)acrylic acid from step b) is at least partly fed into step a).

6. Process according to any of the preceding claims, **characterized in that** the mother liquor removed after step b) is at least partly recycled into step a).

7. Process according to any of the preceding claims, **characterized by** at least two process stages, each of which has steps a) to d), at least one of the following features (α1) to (α4) being satisfied:
(α1) removed (meth) acrylic acid from a first process stage is at least partly fed to a second process stage;
(α2) removed (meth)acrylic acid from a second process stage is at least partly fed to a first process stage;
(α3) mother liquor of a first process stage is at least partly fed to a second process stage;
(α4) mother liquor of a second process stage is at least partly fed to a first process stage.

8. Process according to any of the preceding claims, **characterized by**
(γ1) crystallization of (meth)acrylic acid out of a contaminated crude (meth)acrylic acid stream from a process for preparing (meth)acrylic acid,
(γ2) removal of the (meth)acrylic acid crystals from the mother liquor by means of a scrubbing column, the mother liquor from step being recycled at least partly into step (γ1), and the crude (meth)acrylic acid stream having a purity of < 99.5% by weight of (meth)acrylic acid.

9. Apparatus for preparing (meth)acrylic acid, comprising, as components with a fluid-conducting connection to one another, a (meth)acrylic acid reactor, a quench tower, a distillation apparatus and a purification apparatus which has an apparatus unit which comprises features (δ1) to (δ4)
(δ1) the apparatus unit has a crystallization region, a separation region, a melting apparatus and at least three conduits;
(δ2) the crystallization region is connected to the separation region via a first conduit;
(δ3) the separation region is connected to the melting apparatus via a second conduit;
(δ4) the melting apparatus is connected to the crystallization region via a third conduit.

10. Apparatus according to Claim 9, wherein the apparatus unit has a separate purifying apparatus.

11. Apparatus according to either of Claims 9 and 10, wherein the separation region is connected to the crystallization region by a first recycle line for removed (meth)acrylic acid.

12. Apparatus according to any of Claims 9 to 11, wherein the separation region is connected to the crystallization region by a second recycle line for removed mother liquor.

13. Apparatus according to any of Claims 9 to 12, **characterized by** at least two apparatus units according to features (δ1) to (δ4) which are connected by at least one connecting line, the connecting line being a feed line or a return line, and at least one of the following features (ε1) to (ε4) being satisfied:
(ε1) the separation region of a first apparatus unit is connected via the connecting line to the crystallization region of a second apparatus unit;
(ε2) the melting apparatus of a first apparatus unit is connected via the connecting line to the crystallization region of a second apparatus unit;
(ε3) the separation region of a second apparatus unit is connected via the connecting line to the crystallization region of a first apparatus unit;
(ε4) the melting apparatus of a second apparatus unit is connected via the connecting line to the crystallization region of a first apparatus unit.

14. Apparatus according to any of Claims 9 to 13, wherein the melting apparatus is connected to the separation region via a fourth conduit.

15. Use of an apparatus as defined in any of Claims 9 to 14, or of a purification apparatus as defined in any of Claims 9 to 14, or of a process as defined in any of Claims 1 to 8, for preparation of (meth)acrylic acid having a purity of more than 90% by weight, preferably more than 95% by weight and more preferably more than 99.5% by weight, based in each case on the (meth)acrylic acid with impurities.

## Revendications

1. Procédé de purification d'acide acrylique avec une étape de procédé qui comprend les étapes de procédé suivantes :
a) l'acide acrylique est cristallisé à partir d'une liqueur mère ;
b) l'acide acrylique cristallisé est séparé de la liqueur mère ;
c) au moins une partie des cristaux d'acide acrylique séparés est fondue ; et
d) la partie fondue est recyclée au moins partiellement dans l'étape a) ou dans l'étape b).

2. Procédé de purification d'acide méthacrylique comprenant une étape de procédé qui comprend les étapes de procédé suivantes :
a) l'acide méthacrylique est cristallisé à partir d'une liqueur mère ;
b) l'acide méthacrylique cristallisé est séparé de la liqueur mère ;
c) au moins une partie des cristaux d'acide méthacrylique séparés est fondue ; et
d) la partie fondue est recyclée au moins en partie dans l'étape a) ou dans l'étape b),
l'acide méthacrylique utilisé à l'étape de procédé a) étant un courant d'acide méthacrylique brut qui a été obtenu par distillation d'une solution aqueuse d'acide méthacrylique dans un dispositif de distillation, la solution aqueuse d'acide méthacrylique ayant été obtenue par condensation du mélange d'une oxydation catalytique en phase gazeuse d'isobutène, d'isobutane, de tert-butanol, d'iso-butyraldéhyde, de méthacroléine ou d'éther de méthyle et de tert.-butyle avec de l'oxygène dans une tour de trempe.

3. Procédé selon la revendication 1 ou 2, **caractérisé en ce que** l'acide (méth)acrylique cristallisé est lavé à contre-courant de l'acide (méth)-acrylique recyclé.

4. Procédé selon l'une quelconque des revendications précédentes, **caractérisé en ce que** l'acide (méth)acrylique séparé est purifié lors d'un procédé de purification distinct.

5. Procédé selon l'une quelconque des revendications précédentes, **caractérisé en ce que** l'acide (méth)acrylique cristallisé et séparé issu de l'étape b) est recyclé au moins partiellement dans l'étape a).

6. Procédé selon l'une quelconque des revendications précédentes, **caractérisé en ce que** la liqueur mère séparée selon l'étape b) est recyclée au moins partiellement dans l'étape a).

7. Procédé selon l'une quelconque des revendications précédentes, **caractérisé par** au moins deux étapes de procédé, qui comportent chacune les étapes a) à d), au moins une des caractéristiques (α1) à (α4) suivantes étant satisfaite :
(α1) l'acide (méth)acrylique séparé par une première étape de procédé est introduit au moins partiellement dans une seconde étape de procédé ;
(α2) l'acide (méth)acrylique séparé par une seconde étape de procédé est introduit au moins partiellement dans une première étape de procédé ;
(α3) la liqueur mère d'une première étape de procédé est introduite au moins partiellement dans une seconde étape de procédé ;
(α4) la liqueur mère d'une seconde étape de procédé est introduite au moins partiellement dans une première étape de procédé.

8. Procédé selon l'une quelconque des revendications précédentes, **caractérisé par**
(γ1) la cristallisation d'acide (méth)acrylique à partir d'un courant d'acide (méth)acrylique brut contaminé issu d'un procédé de fabrication d'acide (méth)acrylique,
(γ2) la séparation des cristaux d'acide (méth)acrylique de la liqueur mère au moyen d'une colonne de lavage, la liqueur mère de l'étape étant recyclée au moins en partie dans l'étape (γ1), le courant d'acide (méth)acrylique brut présentant une pureté < 99,5 % en poids en acide (méth)acrylique.

9. Dispositif de fabrication d'acide (méth)acrylique, comprenant, en tant que composants reliés fluidiquement les uns aux autres, u n réacteur d'acide (méth)acrylique, une tour de trempe, un dispositif de distillation et un dispositif de purification, qui comporte une unité de dispositif qui comprend les caractéristiques (δ1) à (δ4)
(δ1) l'unité de dispositif comporte une zone de cristallisation, une zone de séparation, un dispositif de fusion et au moins trois conduites ;
(δ2) la zone de cristallisation est reliée à la zone de séparation par une première conduite ;
(δ3) la zone de séparation est reliée au dispositif de fusion par une deuxième conduite ;
(δ4) le dispositif de fusion est relié à la zone de cristallisation par une troisième conduite.

10. Dispositif selon la revendication 9, dans lequel l'unité de dispositif comporte un dispositif de purification distinct.

11. Dispositif selon l'une quelconque des revendications 9 ou 10, dans lequel la zone de séparation est reliée à la zone de cristallisation par une première conduite de recyclage pour l'acide (méth)acrylique séparé.

12. Dispositif selon l'une quelconque des revendications 9 à 11, dans lequel la zone de séparation est reliée à la zone de cristallisation par une seconde conduite de recyclage pour la liqueur mère séparée.

13. Dispositif selon l'une quelconque des revendications 9 à 12, **caractérisé par** au moins deux unités de dispositif selon les caractéristiques (δ1) à (δ4), qui sont reliées par au moins une conduite de liaison, la conduite de liaison étant une conduite d'alimentation ou une conduite de recyclage, et au moins une des caractéristiques (ε1) à (ε4) suivantes étant satisfaite :
(ε1) la zone de séparation d'une première unité de dispositif est reliée à la zone de cristallisation d'une seconde unité de dispositif par la conduite de liaison ;
(ε2) le dispositif de fusion d'une première unité de dispositif est relié à la zone de cristallisation d'une seconde unité de dispositif par la conduite de liaison ;
(ε3) la zone de séparation d'une seconde unité de dispositif est reliée à la zone de cristallisation d'une première unité de dispositif par la conduite de liaison ;
(ε4) le dispositif de fusion d'une seconde unité de dispositif est relié à la zone de cristallisation d'une première unité de dispositif par la conduite de liaison.

14. Dispositif selon l'une quelconque des revendications 9 à 13, dans lequel le dispositif de fusion est relié à la zone de séparation par une quatrième conduite.

15. Utilisation d'un dispositif tel que défini dans l'une quelconque des revendications 9 à 14 ou d'un dispositif de purification tel que défini dans l'une quelconque des revendications 9 à 14 ou d'un procédé tel que défini dans l'une quelconque des revendications 1 à 8, pour la fabrication d'acide (méth)acrylique, qui présente une pureté supérieure à 90 % en poids, de préférence supérieure à 95 % en poids et de manière particulièrement préférée supérieure à 99,5 % en poids, à chaque fois par rapport à l'acide (méth)acrylique contenant des impuretés.
